# EUROPEAN PATENT APPLICATION

(11) **EP 4 223 782 A1**
(43) Date of publication of application: **09.08.2023**
(21) Application number: 22155486.8
(22) Date of filing: 07.02.2022
(51) Int. Cl.: C07K 16/28, A61P 35/00

(54) **NOVEL T CELL RECEPTORS AND IMMUNE THERAPY USING THE SAME FOR THE TREATMENT OF CANCER**

(71) Applicant: Max-Delbrück-Centrum für Molekulare Medizin in der Helmholtz-Gemeinschaft, 13125 Berlin (DE); Charité - Universitätsmedizin Berlin, 10117 Berlin (DE)
(72) Inventor: BLANKENSTEIN, Thomas, 13092 Berlin (DE); PLEWA, Natalia, 13089 Berlin (DE); PONCETTE, Lucia, 13092 Berlin (DE)
(74) Representative: Kuttenkeuler, David

(57) **Abstract**

The present invention pertains to antigen binding proteins (ABP) which bind to Transforming Growth Factor β Receptor 2 (TGFβR2) derived antigenic peptides. The invention in particular provides novel ABPs, such as T cell receptor (TCR) based molecules, which are selective and specific for an antigenic peptide derived from a frameshift variant of TGFβR2. The TCR of the invention, and ABPs derived therefrom, are of use for the treatment of a proliferative disorder, such as a cancer positive for the expression of TGFβR2 frameshift peptide. Further provided are nucleic acids encoding the antigen binding proteins of the invention, vectors comprising these nucleic acids, recombinant cells expressing antigen binding proteins and pharmaceutical compositions comprising the compounds of the invention.

## Description

The present invention pertains to antigen binding proteins (ABP) which bind to Transforming Growth Factor β Receptor 2 (TGFβR2) derived antigenic peptides. The invention in particular provides novel ABPs, such as T cell receptor (TCR) based molecules, which are selective and specific for an antigenic peptide derived from a frameshift variant of TGFβR2. The TCR of the invention, and ABPs derived therefrom, are of use for the treatment of a proliferative disorder, such as a cancer positive for the expression of TGFβR2 frameshift peptide. Further provided are nucleic acids encoding the antigen binding proteins of the invention, vectors comprising these nucleic acids, recombinant cells expressing antigen binding proteins and pharmaceutical compositions comprising the compounds of the invention.

### BACKGROUND OF THE INVENTION

Transforming growth factor β receptor 2 (TGFβR2) is a serine-threonine protein kinase. This transmembrane receptor binds TGFβ and forms heterodimeric complexes with other receptors and ligand effectively regulating the transcription of many signaling-related genes. TGFβ signaling pathway plays a crucial role in tissue development, cell growth and proliferation, differentiation, apoptosis and homeostasis (Jung *et al.*, 2017). Disruption of those positively or negatively regulated pathways causes many diseases, including cancer. In advanced cancer, a "TGFβ paradox" was observed, highlighting its dual function. Although TGFβ inhibits epithelial cell growth in normal conditions, it promotes tumor cell progression, which further manifests in hallmarks of carcinogenesis like uncontrolled proliferation, epithelial-to-mesenchymal transition, loss of programmed cell death or evasion of the immune system (Principe *et al.*, (2014).

A particular mutation in TGFβR2 caused by a mismatch repair deficiency has been discussed to potentially influence the transformation of normal colonic epithelial cells into malignant ones. Impaired TGFβR2 has been observed to cause lethal inflammatory events like polyps and colon cancer progression in the tumor microenvironment of CRC mediated by TNFα and IL-8 (Principe *et al.*, 2016).

T-cell based immunotherapy targets peptide epitopes derived from tumor-associated or tumor-specific proteins, which are presented by molecules of the major histocompatibility complex (MHC). These tumor associated antigens (TAAs) can be peptides derived from all protein classes, such as enzymes, receptors, transcription factors, etc. which are expressed and, as compared to unaltered cells of the same origin, usually up-regulated in cells of the respective tumor.

Specific elements of the cellular immune response are capable of specifically recognizing and destroying tumor cells. The isolation of T-cells from tumor-infiltrating cell populations or from peripheral blood suggests that such cells play an important role in natural immune defense against cancer. There are two classes of MHC-molecules, MHC class I and MHC class II. Complexes of peptide and MHC class I are recognized by CD8-positive T-cells bearing the appropriate T-cell receptor (TCR), whereas complexes of peptide and MHC class II molecules are recognized by CD4-positive-helper-T-cells bearing the appropriate TCR. Since both types of response, CD8 and CD4 dependent, contribute jointly and synergistically to the anti-tumor effect, the identification and characterization of tumor-associated antigens and corresponding T cell receptors is important in the development of cancer immunotherapies such as vaccines and cell therapies.

A TCR is a heterodimeric cell surface protein of the immunoglobulin super-family, which is associated with invariant proteins of the CD3 complex involved in mediating signal transduction. TCRs exist in αβ and γδ forms, which are structurally similar but have quite distinct anatomical locations and probably functions. The extracellular portion of native heterodimeric aβTCR consists of two polypeptides, each of which has a membrane-proximal constant domain, and a membrane-distal variable domain. Each of the constant and variable domains includes an intra-chain disulfide bond. The variable domains contain the highly polymorphic loops analogous to the complementarity determining regions (CDRs) of antibodies. The use of TCR gene therapy overcomes a number of current hurdles. It allows equipping patients' own T cells with desired specificities and generation of sufficient numbers of T cells in a short period of time, avoiding their exhaustion. The TCR will be transduced into central memory T cells or T cells with stem cell characteristics, which may ensure better persistence and function upon transfer. TCR-engineered T cells will be infused into cancer patients rendered lymphopenic by chemotherapy or irradiation, allowing efficient engraftment but inhibiting immune suppression. In some studies, T cell transfer is also accompanied by IL-2 infusion (Tran et al., 2017).

While advances have been made in the development of molecular-targeting drugs for cancer therapy, there remains a need in the art to develop new anti-cancer agents that specifically target molecules highly specific to cancer cells.

### SUMMARY OF THE INVENTION

According to the present invention, this object is solved by an isolated antigen binding protein (ABP) which specifically binds to a Transforming Growth Factor β Receptor 2 (TGFβR2), derived antigenic peptide, or a variant thereof, wherein the isolated ABP comprises at least one complementarity determining region (CDR) being a CDR3 having an amino acid sequence with at least 80% sequence identity to, or having no more than three or two, preferably no more than one amino acid substitution(s), deletion(s) or insertion(s) compared to, a sequence selected from SEQ ID Nos: 11, 13, 15, and 17.

According to the present invention, this object is solved by an isolated nucleic acid encoding for an ABP of the present invention.

According to the present invention, this object is solved by a recombinant vector comprising a nucleic acid of the present invention.

According to the present invention, this object is solved by a recombinant host cell comprising an ABP of the present invention, or a nucleic acid of the present invention, or a vector of the present invention.

According to the present invention, this object is solved by a pharmaceutical composition comprising the ABP of the present invention, or a nucleic acid of the present invention, or a vector of the present invention, or the host cell of the present invention, and a pharmaceutical acceptable carrier, stabilizer and/or excipient.

According to the present invention, this object is solved by a compound or composition for use in medicine, wherein the compound or composition is selected from an ABP of the present invention, or a nucleic acid of the present invention, or a vector of the present invention, or a host cell of the present invention, or a pharmaceutical composition of the present invention.

According to the present invention, this object is solved by a compound or composition for use in the treatment of a proliferative disorder, preferably cancer,
wherein the compound or composition is selected from an ABP of the present invention, or a nucleic acid of the present invention, or a vector of the present invention, or a host cell of the present invention, or a pharmaceutical composition of the present invention.

According to the present invention, this object is solved by a method of manufacturing a TGFβR2 frameshift peptide-specific antigen recognizing construct expressing cell line, preferably a TGFβR2(-1) frameshift peptide-specific antigen recognizing construct expressing cell line, comprising
(a) providing a suitable host cell,
(b) providing a genetic construct comprising a coding sequence encoding the ABP according to the present invention,
(c) introducing into said suitable host cell said genetic construct, and
(d) expressing said genetic construct by said suitable host cell.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 21" should be interpreted to include not only the explicitly recited values of 1 to 21, but also include individual values and sub-ranges within the indicated range. Thus, included in this numerical range are individual values such as 1, 2, 3, 4, 5 .... 17, 18, 19, 20, 21 and sub-ranges such as from 2 to 10, 8 to 15, etc. This same principle applies to ranges reciting only one numerical value, such as "at least 80%". Furthermore, such an interpretation should apply regardless of the breadth of the range or the characteristics being described.

### Antigen binding proteins (ABP)

As outlined above, the present invention provides an isolated antigen binding protein (ABP).

An isolated antigen binding protein (ABP) of the present invention specifically binds to a Transforming Growth Factor β Receptor 2 (TGFβR2), preferably a mutated TGFβR2, derived antigenic peptide, or a variant thereof.

Said isolated ABP comprises at least one complementarity determining region (CDR) being a CDR3 having an amino acid sequence with at least 80% sequence identity to, or having no more than three or two, preferably no more than one amino acid substitution(s), deletion(s) or insertion(s) compared to, a sequence selected from SEQ ID Nos: 11, 13, 15, and 17.

Preferably, the ABP of the present invention comprises a CDR3 having at least 80%, 90%, 95%, 98%, 99%, or preferably 100% sequence identity to an amino acid sequence selected from SEQ ID Nos. 11, 13, 15, and 17.

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site). In a particular embodiment, for example when comparing the protein or nucleic acid sequence of an antigen binding protein of the invention to another protein/gene, the percentage identity can be determined by the Blast searches; in particular for amino acid identity, those using BLASTP 2.2.28+ with the following parameters: Matrix: BLOSUM62; Gap Penalties: Existence: 11, Extension: 1; Neighboring words threshold: 11; Window for multiple hits: 40.

In the context of the present invention it shall be understood that any embodiments referred to as "comprising" certain features of the invention, shall be understood to include in some more preferred embodiments the more restricted description of "consisting of' or "consisting essentially of' the very same features of the present invention.

In another additional or alternative embodiment, the antigen binding protein (ABP) may further comprise a CDR1 and/or a CDR2 domain sequence. Within the variable domain, CDR1 and CDR2 are found in the variable (V) region of a polypeptide chain, and CDR3 includes some of V, all of diversity (D) and joining (J) regions. CDR3 is the most variable and is the main CDR responsible for specifically and selectively recognizing an antigen. CDR1 and CDR2 sequences may be selected from a CDR sequence of a human variable chain allele.

Native alpha-beta heterodimeric TCRs have an alpha chain and a beta chain. Each chain comprises variable, joining and constant regions, and the beta chain also usually contains a short diversity region between the variable and joining regions, but this diversity region is often considered as part of the joining region. Each variable region comprises three CDRs (Complementarity Determining Regions) embedded in a framework sequence, one being the hypervariable region named CDR3. There are several types of alpha chain variable (Vα) regions and several types of beta chain variable (Vβ) regions distinguished by their framework, CDR1 and CDR2 sequences, and by a partly defined CDR3 sequence. The Vα types are referred to in IMGT nomenclature by a unique TRAV number, Vβ types are referred to by a unique TRBV number. For more information on immunoglobulin antibody and TCR genes see the international ImMunoGeneTics information system^{®}, Lefranc M-P et al (Nucleic Acids Res. 2015 Jan;43(Database issue):D413-22; and http://www.imgt.org/).

Therefore, in one additional or alternative embodiment the antigen binding protein (ABP) of the invention comprises CDR1, CDR2 and CDR3 sequences in a combination. Therefore, preferred are antigen binding proteins of the invention which comprise at least one, preferably, all three CDR sequences CDR1, CDR2 and CDR3. Preferably, an antigen binding protein of the invention comprises the respective CDR1 to CDR3 of one individual herein disclosed TCR variable region of the invention (see details below and the example section).

The term "specificity" or "antigen specificity" or "specific for" a given antigen, as used herein means that the antigen binding protein can specifically bind to said antigen, preferably with high avidity, when said antigen is presented by HLA, preferably by HLA type DR4. For example, a TCR, as antigen binding protein, may be considered to have "antigenic specificity" for the antigenic peptide, if T cells expressing the TCR and contacted with an antigenic peptide-presenting HLA secrete at least about 200 pg/ml or more (e.g., 250 pg/ml or more, 300 pg/ml or more, 400 pg/ml or more, 500 pg/ml or more, 600 pg/ml or more, 700 pg/ml or more, 1000 pg ml or more, 2,000 pg/ml or more, 2,500 pg/ml or more, 5,000 pg/ml or more) of interferon γ (IFN-γ) upon co-culture with target cells pulsed with a low concentration of an antigenic peptide of the invention (i.e. the antigenic peptide derived from a frameshift variant of TGFβR2), such as the antigenic peptides provided herein below (e.g., about 10-11 mol/l, 10-10 mol/l, 10-9 mol/l, 10-8 mol/l, 10-7 mol/l, 10-6 mol/l, 10-5 mol/l). Alternatively, or additionally, a TCR may be considered to have "antigenic specificity" for the antigenic peptide, if T cells expressing the TCR secrete at least twice as much IFN-γ as the untransduced background level of IFN-γ upon co-culture with target cells pulsed with a low concentration of the antigenic peptides. Such a "specificity" as described above can - for example - be analyzed with an ELISA.

In a preferred embodiment, the ABP of the present invention is capable of specifically and/or selectively binding to an antigenic peptide derived from a frameshift variant of TGFβR2.

The term "selectivity" or "selective recognizing/binding" is understood to refer to the property of an antigen binding protein (such as a TCR or antibody) to selectively recognize or bind to preferably only one specific epitope and preferably shows no or substantially no cross-reactivity to another epitope. Preferably "selectivity" or "selective recognizing/binding" means that the antigen binding protein (e.g. a TCR) selectively recognizes or binds to preferably only one specific epitope and preferably shows no or substantially no cross-reactivity to another epitope, wherein said epitope is unique for one protein, such that the antigen binding protein shows no or substantially no cross-reactivity to another epitope and another protein.

Transforming growth factor β receptor 2 (TGFβR2) is a serine-threonine protein kinase. The human gene coding TGFβR2 protein has 10-adenine repeats (microsatellite) from base number 709 to 718, located in exon 3 of the gene (Markowitz *et al.*, (1995). Somatic non-synonymous mutations in this polyadenine sequences have been observed: deletion (TGFβR2(-1)) or addition (TGFβR2(+1)) of one adenine (Saeterdal *et al.*, 2001). Deletion or addition of one nucleotide (-1 or +1) results in a new nucleotide triplet coding new amino acids from base position 128 or 129 and, in the end, a new frameshift variant of the peptide. The TGFβR2(+1) frameshift gives almost an immediate termination of the peptide sequence (the stop codon comes as a fourth amino acid after the +1A mutation). The TGFβR2(-1) mutation results in a 34 amino acids sequence that serves as a potential source of unique immunogenic neoantigens. TGFβR2(-1) neoantigen is present in ~77% of MSI-CRC patients (Tougeron *et al.*, 2009; Schwitalle *et al.*, 2008; Maby *et al.*, 2015). Such high frequency of this new class, shared neoantigen (public neoantigen), allows to treat more patients with the same immunotherapy, as opposed to an expensive tailored individualized approach.

Preferably, the frameshift variant of TGFβR2 is derived from a sequence comprising a frame-shift mutation in the wild type TGFβR2 gene sequence according to SEQ ID No: 1 or 2.

More preferably, the frameshift mutation in TGFβR2 is a deletion or an addition of one nucleotide. The position of the mutation is preferably less relevant.

In a preferred embodiment, the frameshift mutation is a deletion or an addition of one nucleotide in a microsatellite region of the TGFβR2 gene sequence according to SEQ ID No: 1 or 2.

More preferably, the microsatellite region is a polyadenine sequence, such as a repeat of adenines located in exon 3 of the wild type TGFβR2 gene sequence according to SEQ ID No: 1 or 2.

For example, the microsatellite region is a repeat of adenines within the sequence comprising nucleotide numbers 709 to 718 of the wild type TGFβR2 gene sequence according to SEQ ID No: 1 or 2.

Preferably, the frameshift mutation is a deletion or an addition of one adenine.

Preferably, the frameshift mutation causes a shift of the open reading frame of the wild type TGFβR2 gene sequence according to SEQ ID No: 1 or 2 due to the deletion or addition of one nucleotide.

More preferably, the frameshift variant of TGFPR2 comprises an amino acid at position 128 or 129 according to the numbering in the wild type TGFβR2 amino acid sequence according to SEQ ID No: 5 or 6 that differs from the amino acid at position 128 or 129 of the wild type TGFβR2 amino acid sequence according to SEQ ID No: 5 or 6.

More preferably, the frameshift variant of TGFβR2 comprises an amino acid sequence according to SEQ ID No: 10.

Preferably, the frameshift mutation is a deletion of one nucleotide within the sequence comprising nucleotide numbers 709 to 718 of the wild type TGFβR2 gene sequence according to SEQ ID No: 1 or 2. More preferably, the deletion of one nucleotide is a deletion of one adenine.

In a preferred embodiment, the ABP is an antibody, or an antigen binding derivative or fragment thereof, or, preferably, is a T cell receptor (TCR), or an antigen binding derivative or fragment thereof.

The ABP, or the binding functionality of the TCR of the invention, may be provided in the framework of an antibody. For example CDR sequences of the TCR of the invention, possibly including additional 3, 2 or 1 N- and/or C-terminal framework residues, may be directly grafted into an antibody variable heavy/light chain sequence. The term "antibody" in its various grammatical forms is used herein to refer to immunoglobulin molecules and immunologically active portions of immunoglobulin molecules, i.e., molecules that contain an antigen-binding site or a paratope. Such molecules are also referred to as "antigen binding fragments" of immunoglobulin molecules. The invention further provides an antibody, or antigen binding portion thereof, which specifically binds to the antigens described herein. The antibody can be any type of immunoglobulin that is known in the art. For instance, the antibody can be of any isotype, e.g., IgA, IgD, IgE, IgG, IgM, etc. The antibody can be monoclonal or polyclonal. The antibody can be a naturally-occurring antibody, e.g., an antibody isolated and/or purified from a mammal, e.g., mouse, rabbit, goat, horse, chicken, hamster, human, etc. Alternatively, the antibody can be a genetically-engineered antibody, e.g., a humanized antibody or a chimeric antibody or a murinized antibody. The antibody can be in monomeric or polymeric form.

The term "antibody" includes, but is not limited to, genetically engineered or otherwise modified forms of immunoglobulins, such as intrabodies, chimeric antibodies, fully human antibodies, humanized antibodies (e.g. generated by "CDR-grafting"), antibody fragments, and heteroconjugate antibodies (e.g., bispecific antibodies, diabodies, triabodies, tetra-bodies, etc.). The term "antibody" includes cys-diabodies and minibodies. Thus, each and every embodiment provided herein in regard to "antibodies", or "antibody like constructs" is also envisioned as, bi-specific antibodies, diabodies, scFv fragments, chimeric antibody receptor (CAR) constructs, diabody and/or minibody embodiments, unless explicitly denoted otherwise. The term "antibody" includes a polypeptide of the immunoglobulin family or a polypeptide comprising fragments of an immunoglobulin that is capable of non-covalently, reversibly, and in a specific manner binding a corresponding antigen, preferably an antigenic peptide of the invention, i.e. the antigenic peptide derived from a frameshift variant of TGFβR2, as disclosed herein. An exemplary antibody structural unit comprises a tetramer. In some embodiments, a full length antibody can be composed of two identical pairs of polypeptide chains, each pair having one "light" and one "heavy" chain (connected through a disulfide bond). Antibody structure and isotypes are well known to the skilled artisan (for example from Janeway's Immunobiology, 9th edition, 2016).

The recognized immunoglobulin genes of mammals include the kappa, lambda, alpha, gamma, delta, epsilon, and mu constant region genes, as well as the myriad immunoglobulin variable region genes (for more information on immunoglobulin genes see the international ImMunoGeneTics information system^{®}, Lefranc M-P et al, Nucleic Acids Res. 2015 Jan;43(Database issue):D413-22; and http://www.imgt.org/). For full-length chains, the light chains are classified as either kappa or lambda. For full-length chains, the heavy chains are classified as gamma, mu, alpha, delta, or epsilon, which in turn define the immunoglobulin classes, IgG, IgM, IgA, IgD, and IgE, respectively. The N-terminus of each chain defines a variable region of about 100 to 110 or more amino acids primarily responsible for antigen recognition. The terms variable light chain (VL) and variable heavy chain (VH) refer to these regions of light and heavy chains respectively. As used in this invention, an "antibody" encompasses all variations of antibody and fragments thereof. Thus, within the scope of this concept are full length antibodies, chimeric antibodies, humanized antibodies, single chain antibodies (scFv), Fab, Fab', and multimeric versions of these fragments (e.g., F(ab')2) with the same, essentially the same or similar binding specificity. In some embodiments, the antibody binds specifically to an antigenic peptide of the invention, i.e. the antigenic peptide derived from a frameshift variant of TGFβR2. Preferred antigen binding proteins according to the invention include an antibody heavy chain, preferably the variable domain thereof, or an antigen binding fragment thereof, and/or an antibody light chain, preferably the variable domain thereof, or an antigen binding fragment thereof. Similarly, disulfide-stabilized variable region fragments (dsFv) can be prepared by recombinant DNA technology, antibody fragments of the invention, however, are not limited to these exemplary types of antibody fragments. Also, the antibody, or antigen binding portion thereof, can be modified to comprise a detectable label, such as, for instance, a radioisotope, a fluorophore (e.g., fluorescein isothiocyanate (FITC), phycoerythrin (PE)), an enzyme (e.g., alkaline phosphatase, horseradish peroxidase), and element particles (e.g., gold particles). In some instances, the TCR CDR3 sequence may be slightly modified, but preferably by not more than 3 amino acid residues, preferably only two and most preferably only one amino acid position, as compared to the CDR3 sequences provided in SEQ ID Nos: 11, 13, 15 and 17. Preferably, the antibodies comprise the CDR3, preferably all of CDR1 to CDR3 regions in the combination, in each case independently, optionally with not more than three or two, preferably one, amino acid substitution(s), insertion(s) and/or deletion(s) compared to these sequences.

Suitable methods of making antibodies are known in the art. For instance, standard hybridoma methods are described in, e.g., Kohler and Milstein, Eur. J. Immunol, 5, 51 1-519 (1976), Harlow and Lane (eds.), Antibodies: A Laboratory Manual, CSH Press (1988), and C.A. Janeway et al. (eds.), Immunobiology, 8 Ed., Garland Publishing, New York, NY (201 1)). Alternatively, other methods, such as EBV-hybridoma methods (Haskard and Archer, J. Immunol. Methods, 74(2), 361-67 (1984), and Roder et al, Methods Enzymol, 121, 140-67 (1986)), and bacteriophage vector expression systems (see, e.g., Huse et al., Science, 246, 1275-81 (1989)) are known in the art. Further, methods of producing antibodies in non-human animals are described in, e.g., U.S. Patents 5,545,806, 5,569,825, and 5,714,352, and U.S. Patent Application Publication No. 2002/0197266.

Preferably, the ABP of the present invention binds to a human leucocyte antigen (HLA) presented antigenic peptide, wherein said HLA is more preferably a HLA type DR4. Preferably, the ABP forms a specific complex with the MHC and the antigenic peptide.

Preferably, the ABP of the present invention is humanized, chimerized and/or murinized.

Preferably, the ABP of the present invention specifically and/or selectively binds to an epitope having the amino acid sequence selected from an MHC presented peptide of a frameshift variant of TGFβR2, more preferably the peptide comprises a frame-shift mutation in TGFβR2, and most preferably a peptide comprising SEQ ID No: 10.

Preferably, the ABP of the present invention is an α/β-TCR, or an antigen binding derivative or a fragment thereof, or wherein the ABP is a γ/δ-TCR, or an antigen binding derivative or a fragment thereof.

Preferably, the ABP of the present invention is of human origin and specifically and/or selectively recognizes a TGFβR2 frameshift antigenic peptide.

Preferably, the ABP of the present invention is capable of inducing an immune response in a subject, optionally wherein the immune response is characterized by an increase in interferon (IFN) γ levels.

TCRs of the invention may be provided as single chain α or β, or γ and δ, molecules, or alternatively as double chain constructs composed of both the α and β chain, or γ and δ chain. The ABP of the invention may comprise a TCR α or γ chain; and/or a TCR β or δ chain;
wherein the TCR α or γ chain comprises a CDR3 having an amino acid sequence with at least 50%, 60%, 70%, 80%, 90%, 95%, 98%, 99%, or 100% sequence identity to, or having no more than three or two, preferably no more than one amino acid substitution(s), deletion(s) or insertion(s) compared to, a sequence selected from SEQ ID Nos: 11 and 15;
and/or wherein the TCR β or δ chain comprises a CDR3 having at least 50%, 60%, 70%, 80%, 90%, 95%, 98%, 99%, or 100% sequence identity to, or having no more than three or two, preferably no more than one amino acid substitution(s), deletion(s) or insertion(s) compared to, a sequence selected from SEQ ID Nos: 13 and 17.

Preferably, the ABP of the present invention comprises a TCR variable chain region having at least 50%, 60%, 70%, 80%, 90%, 95%, 98%, 99%, or 100% sequence identity to, or having no more than 20, preferably no more than 15, more preferably no more than 10, 5, three or two, preferably no more than one amino acid substitution(s), deletion(s) or insertion(s) compared to, an amino acid sequence selected from SEQ ID Nos. 12, 14, 16, and 18.

Preferably, the ABP of the present invention comprises a binding fragment of a TCR, and wherein said binding fragment comprises a CDR1 to CDR3, optionally selected from the CDR1 to CDR3 sequences having the amino acid sequences of SEQ ID Nos. 11, 13, 15, and 17; each CDR independently having no more than three or two, preferably no more than one amino acid substitution(s), deletion(s) or insertion(s) compared to these sequences.

Preferably, the ABP of the present invention is a TCR, or an antigen-binding fragment or derivative thereof, comprising at least one TCR α and one TCR β chain sequence,
wherein
   said TCR α chain sequence comprises a variable region sequence having the amino acid sequence of SEQ ID No: 12, and
   said TCR β chain sequence comprises a variable region sequence having the amino acid sequence of SEQ ID No: 14;
or wherein
   said TCR α chain sequence comprises a variable region sequence having the amino acid sequence of SEQ ID No: 16, and
   said TCR β chain sequence comprises a variable region sequence having the amino acid sequence of SEQ ID No: 18;
each variable domain sequence having no more than 20, preferably no more than 15, more preferably no more than 10, 5, three or two, preferably no more than one amino acid substitution(s), deletion(s) or insertion(s) compared to these sequences.

Preferably, the ABP of the present invention is a TCR, or an antigen-binding fragment or derivative thereof, further comprising a TCR constant region, preferably a human TCR constant region sequence.

The inventive TCRs may further comprise a constant region derived from any suitable species, such as any mammal, e.g., human, rat, monkey, rabbit, donkey, or mouse. In an embodiment of the invention, the inventive TCRs further comprise a human constant region. In some preferred embodiments, the constant region of the TCR of the invention may be slightly modified, for example, by the introduction of heterologous sequences, preferably mouse sequences, which may increase TCR expression and stability.

As used herein, the term "murine" or "human," when referring to an ABP, or a TCR, or any component of a TCR described herein (e.g., complementarity determining region (CDR), variable region, constant region, α chain, and/or β chain), means a TCR (or component thereof), which is derived from a mouse or a human unrearranged TCR locus, respectively.

In an embodiment of the invention, chimeric TCR are provided, wherein the TCR chains comprise sequences from multiple species. Preferably, a TCR of the invention may comprise an α chain comprising a human variable region of an α chain and, for example, a murine constant region of a murine TCR α chain.

In one embodiment, the TCR of the invention is a human TCR comprising human variable regions according to the above embodiments and human constant regions.

In some embodiments the ABP is murinized or humanized. These terms are used when amino acid sequences from a foreign species are introduced into a construct of the invention.

The TCR of the invention may be provided as a single chain TCR (scTCR). Preferably a scTCR of the invention comprises a variable region of an alpha chain and a variable region of a beta chain in one genetic construct - as one protein polymer. Therefore, the scTCR of the invention comprises two variable regions connected by a linker. The linker may either directly connect the two variable regions, or connect the two variable regions via constant domains located in between. Some scTCR constructs therefore may pertain to two alpha beta chain sequences connected by a linker. A scTCR may comprise a polypeptide of a variable region of a first TCR chain (e.g., an alpha chain) and a polypeptide of an entire (full-length) second TCR chain (e.g., a beta chain), or vice versa. Furthermore, the scTCR can optionally comprise one or more linkers which join the two or more polypeptides together. The linker can be, for instance, a peptide, which joins together two single chains, as described herein. Also provided is such a scTCR of the invention, which is fused to a human cytokine, such as IL-2, IL-7 or IL-15.

The ABP according to the invention can also be provided in the form of a multimeric complex, comprising at least two scTCR molecules, wherein said scTCR molecules are each fused to at least one biotin moiety, or other interconnecting molecule/linker, and wherein said scTCRs are interconnected by biotin-streptavidin interaction to allow the formation of said multimeric complex. Similar approaches known in the art for the generation of multimeric TCR are also possible and included in this disclosure. Also provided are multimeric complexes of a higher order, comprising more than two scTCR of the invention.

For the purposes of the present invention, a TCR is a moiety having at least one TCR alpha or gamma and/or TCR beta or delta variable domain. Generally, they comprise both a TCR alpha variable domain and a TCR beta variable domain, alternatively both a TCR gamma variable domain and a TCR delta variable domain. They may be αβ/γδ heterodimers or may be in single chain format. For use in adoptive therapy, an αβ or γδ heterodimeric TCR may, for example, be transfected as full length chains having both cytoplasmic and transmembrane domains. If desired, an introduced disulfide bond between residues of the respective constant domains may be present.

In a preferred embodiment, the ABP is a human TCR, or fragment or derivative thereof. A human TCR or fragment or derivative thereof is a TCR, which comprises over 50% of the corresponding human TCR sequence. Preferably, only a small part of the TCR sequence is of artificial origin or derived from other species. It is known, however, that chimeric TCRs, e.g. derived from human origin with murine sequences in the constant domains, are advantageous. Particularly preferred are, therefore, TCRs in accordance with the present invention, which contain murine sequences in the extracellular part of their constant domains.

Thus, it is also preferred that the inventive ABP is able to recognize its antigen in a human leucocyte antigen (HLA) dependent manner, preferably in a HLA-DR4 dependent manner. The term "HLA dependent manner" in the context of the present invention means that the antigen recognizing construct binds to the antigen only in the event that the antigenic peptide is presented by said HLA.

The ABP in accordance with the invention in one embodiment preferably induces an immune response, preferably wherein the immune response is characterized by the increase in interferon (IFN) γ levels.

### Nucleic acids, vectors and cell lines

As outlined above, the present invention provides an isolated *nucleic acid* encoding for an antigen binding protein of the present invention, or any of the aforementioned protein or polypeptide constructs.

The nucleic acid preferably (a) has a strand encoding for an antigen binding protein according to the invention; (b) has a strand complementary to the strand in (a); or (c) has a strand that hybridizes under stringent conditions with a molecule as described in (a) or (b). Stringent conditions are known to the person of skill in the art, specifically from Sambrook et al, "Molecular Cloning". In addition to that, the nucleic acid optionally has further sequences, which are necessary for expressing the nucleic acid sequence corresponding to the protein, specifically for expression in a mammalian/human cell. The nucleic acid used can be contained in a vector suitable for allowing expression of the nucleic acid sequence corresponding to the peptide in a cell. However, the nucleic acids can also be used to transform an antigen-presenting cell, which may not be restricted to classical antigen-presenting cells, such as dendritic cells, in such a way that they themselves produce the corresponding proteins on their cellular surface.

In some embodiments, the polypeptides of the antigen binding proteins can be encoded by nucleic acids and expressed *in vivo* or *in vitro.* Thus, in some embodiments, a nucleic acid encoding an antigen binding proteinis provided. In some embodiments, the nucleic acid encodes one part or monomer of an antigen binding protein of the invention (for example one of two chains of a TCR of the invention), and/or another nucleic acid encodes another part or monomer of an antigen binding proteinof the invention (for example the other of two chains of the TCR). In some embodiments, the nucleic acid encodes two or more antigen binding protein polypeptide chains, for example, at least 2 TCR chains. Nucleic acids encoding multiple antigen binding protein chains can include nucleic acid cleavage sites between at least two chain sequences, can encode transcription or translation start site between two or more chains sequences, and/or can encode proteolytic target sites between two or more antigen binding protein chains.

By "nucleic acid" as used herein includes "polynucleotide," "oligonucleotide," and "nucleic acid molecule," and generally means a polymer of DNA or RNA, which can be single-stranded or double-stranded, synthesized or obtained (e.g., isolated and/or purified) from natural sources, which can contain natural, non-natural or altered nucleotides, and can contain a natural, non-natural or altered internucleotide linkage, such as a phosphoroamidate linkage or a phosphorothioate linkage, instead of the phosphodiester found between the nucleotides of an unmodified oligonucleotide.

Preferably, the nucleic acids of the invention are recombinant. As used herein, the term "recombinant" refers to (i) molecules that are constructed outside living cells by joining natural or synthetic nucleic acid segments to nucleic acid molecules that can replicate in a living cell, or (ii) molecules that result from the replication of those described in (i) above. For purposes herein, the replication can be *in vitro* replication or *in vivo* replication. The nucleic acid can comprise any nucleotide sequence, which encodes any of the TCRs, polypeptides, or proteins, or functional portions or functional variants thereof described herein.

The isolated nucleic acid of present invention comprises a nucleic acid sequence having at least 80% sequence identity to, or having no more than 20, preferably no more than 15, more preferably no more than 10, 5, three or two, preferably no more than one nucleic acid substitution(s), deletion(s) or insertion(s) compared to, a nucleic acid sequence encoding for a polypeptide selected from SEQ ID Nos: 12, 14, 16, and 18.

Preferably, the nucleic acid of present invention comprises a nucleic acid sequence having at least 80%, 90%, 95%, 98%, 99%, or preferably 100% sequence identity to a nucleic acid sequence encoding for a polypeptide selected from SEQ ID Nos: 12, 14, 16, and 18.

As outlined above, the present invention provides a recombinant *vector* comprising a nucleic acid of the present invention.

Desirably, the vector is an expression vector or a recombinant expression vector. The term "recombinant expression vector" refers in context of the present invention to a nucleic acid construct that allows for the expression of an mRNA, protein or polypeptide in a suitable host cell. The recombinant expression vector of the invention can be any suitable recombinant expression vector, and can be used to transform or transfect any suitable host. Suitable vectors include those designed for propagation and expansion or for expression or both, such as plasmids and viruses. Examples of animal expression vectors include pEUK-Cl, pMAM, and pMAMneo. Preferably, the recombinant expression vector is a viral vector, e.g., a retroviral vector. The recombinant expression vector comprises regulatory sequences, such as transcription and translation initiation and termination codons, which are specific to the type of host cell (e.g., bacterium, fungus, plant, or animal), into which the vector is to be introduced and in which the expression of the nucleic acid of the invention may be performed. Furthermore, the vector of the invention may include one or more marker genes, which allow for selection of transformed or transfected hosts. The recombinant expression vector can comprise a native or normative promoter operably linked to the nucleotide sequence encoding the constructs of the invention, or to the nucleotide sequence, which is complementary to or which hybridizes to the nucleotide sequence encoding the proteins of the invention. The selections of promoters include, e.g., strong, weak, inducible, tissue-specific and developmental-specific promoters. The promoter can be a non-viral promoter or a viral promoter. The inventive recombinant expression vectors can be designed for either transient expression, for stable expression, or for both. Also, the recombinant expression vectors can be made for constitutive expression or for inducible expression.

As outlined above, the present invention provides a recombinant *host cell* comprising an ABP of the present invention, or a nucleic acid of the present invention, or a vector of the present invention.

The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension.

For purposes of producing a recombinant TCR, polypeptide, or protein, the host cell is preferably a mammalian cell. Most preferably, the host cell is a human cell. While the host cell can be of any cell type, can originate from any type of tissue, and can be of any developmental stage, the host cell preferably is a peripheral blood leukocyte (PBL) or a peripheral blood mononuclear cell (PBMC), preferably a lymphocyte. More preferably, the host cell is a T cell. The T cell can be any T cell, such as a cultured T cell, e.g., a primary T cell, or a T cell from a cultured T cell line, e.g., Jurkat, SupTi, etc., or a T cell obtained from a mammal, preferably a T cell or T cell precursor from a human patient. If obtained from a mammal, the T cell can be obtained from numerous sources, including but not limited to blood, bone marrow, lymph node, the thymus, or other tissues or fluids. T cells can also be enriched for or purified. Preferably, the T cell is a human T cell. More preferably, the T cell is a T cell isolated from a human. The T cell can be any type of T cell and can be of any developmental stage, including but not limited to, CD4-positive and/or CD8-positive, CD4-positive helper T cells, e.g., Thi and Th2 cells, CD8-positive T cells (e.g., cytotoxic T cells), tumor infiltrating cells (TILs), memory T cells, naive T cells, and the like. Most preferably, the T cell is a CD4-positive T cell.

Preferably, the host cell is a lymphocyte, more preferably a T lymphocyte or T lymphocyte progenitor, even more preferably a CD4 or a CD8 positive T-cell, most preferably a CD4 positive T-cell.

### Methods of manufacturing cell lines

As outlined above, the present invention provides a method of manufacturing a TGFβR2 frameshift peptide-specific antigen recognizing construct expressing cell line, preferably a TGFβR2(-1) frameshift peptide-specific antigen recognizing construct expressing cell line.

Said method comprises the steps:
(a) providing a suitable host cell,
(b) providing a genetic construct comprising a coding sequence encoding the antigen binding protein according to the herein discloses invention,
(c) introducing into said suitable host cell said genetic construct, and
(d) expressing said genetic construct by said suitable host cell.

The method may further comprise a step of cell surface presentation of said antigen recognizing construct on said suitable host cell.

In other preferred embodiments, the genetic construct is an expression construct comprising a promoter sequence operably linked to said coding sequence.

Preferably, said antigen binding protein is of mammalian origin, preferably of human origin. The preferred suitable host cell for use in the method of the invention is a mammalian cell, such as a human cell, in particular a human T lymphocyte. T cells for use in the invention are described in detail herein above.

Also encompassed by the invention are embodiments, wherein said antigen binding protein is a modified TCR, wherein said modification is the addition of functional domains, such as a label or a therapeutically active substance. Furthermore, encompassed are TCR having alternative domains, such as an alternative membrane anchor domain instead of the endogenous transmembrane region.

Preferably, the ABP is an alpha/beta TCR, gamma/delta TCR, or a TCR variant, such as a single chain TCR (scTCR).

Desirably, the transfection system for introducing the genetic construct into said suitable host cell is a retroviral vector system. Such systems are well known to the skilled artisan.

Also comprised by the present invention is in one embodiment the additional method step of isolation and purification of the antigen binding protein from the cell and, optionally, the reconstitution of the translated antigen binding protein in a T-cell.

In a preferred embodiment, the suitable host cell is a cell, such as a T-cell, obtained from a patient suffering from a disorder associated with a TGFβR2 frameshift peptide expression, preferably with a TGFβR2(-1) frameshift peptide expression, such as more preferably a cancer, for example colorectal cancer (CRC).

The term "isolated" as used herein in the context of a polypeptide, such as an antigen binding protein (an example of which could be an antibody), refers to a polypeptide that is purified from proteins or polypeptides or other contaminants that would interfere with its therapeutic, diagnostic, prophylactic, research or other use. An antigen binding protein according to the invention may be a recombinant, synthetic or modified (non-natural) antigen binding protein. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. In this context, a "recombinant" protein/polypeptide or nucleic acid is one made using recombinant techniques. Methods and techniques for the production of recombinant nucleic acids and proteins are well known in the art.

### Pharmaceutical compositions

As outlined above, the present invention provides *a pharmaceutical composition* comprising:
the ABP of the present invention, or a nucleic acid of the present invention, or a vector of the present invention, or the host cell of the present invention, and
a pharmaceutical acceptable carrier, stabilizer and/or excipient.

Preferably, the pharmaceutical composition comprises at least two different ABP of the present invention.

A pharmaceutical composition of the present invention comprises any of the herein described products of the invention and TCR materials of the invention, specifically any proteins, nucleic acids or host cells. In a preferred embodiment the pharmaceutical composition is for immune therapy, preferably adoptive cell therapy.

Preferably, the pharmaceutical composition is administered by injection, e.g., intravenously. When the pharmaceutical composition comprises a host cell expressing the inventive ABP (or functional variant thereof), the pharmaceutically acceptable carrier for the cells for injection may include any isotonic carrier such as, for example, normal saline (about 0.90% w/v of NaCl in water, about 300 mOsm/L NaCl in water, or about 9.0 g NaCl per liter of water), NORMOSOL R electrolyte solution (Abbott, Chicago, IL), PLASMA-LYTE A (Baxter, Deerfield, IL), about 5% dextrose in water, or Ringer's lactate. In an embodiment, the pharmaceutically acceptable carrier is supplemented with human serum albumen.

For purposes of the invention, the amount or dose (e.g., numbers of cells when pharmaceutical composition comprises cells) of the inventive ABP administered may be sufficient to affect, e.g., a therapeutic or prophylactic response, in the subject or animal over a reasonable time frame. For example, the dose should be sufficient to bind to a cancer antigen, or detect, treat or prevent cancer in a period of from about 2 hours or longer, e.g., 12 to 24 or more hours, from the time of administration. In certain embodiments, the time period could be even longer. The dose will be determined by the efficacy of the particular inventive ABP and the condition of the animal (e.g., human), as well as the body weight of the animal (e.g., human) to be treated.

Examples of pharmaceutically acceptable carriers or diluents useful in the present invention include stabilizers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer).

### Medical uses and treatment methods

As outlined above, the present invention provides the herein disclosed antigen binding proteins, nucleic acids, vectors, pharmaceutical compositions and/or host cell for use in medicine.

The use in medicine in one preferred embodiment includes the use in the treatment of a proliferative disorder, preferably cancer.

The cancer is in particular positive for the expression of a TGFβR2 frameshift peptide.

With respect to the above mentioned medical applications of the antigen binding proteins and other materials derived therefrom, pertaining thereto or encoding the same, in accordance of the present disclosure, the diseases to be treated can be any proliferative disorder, preferably characterized by the expression of a TGFβR2 frameshift peptide.

A preferred cancer is colorectal cancer (CRC) or any cancer with microsatellite instability or any cancer that bears a TGFBR2(-1) mutation.

Preferably, the treatment comprises immune therapy, preferably adoptive autologous or heterologous T-cell therapy.

The antigen binding proteins (such as TCRs antibodies, polypeptides), nucleic acids, vectors, pharmaceutical compositions and/or host cells of the invention are in particular for use in immune therapy, preferably, in adoptive T cell therapy.

The administration of the compounds and compositions of the invention can, for example, involve the infusion of T cells of the invention into said patient. Preferably, such T cells are autologous T cells of the patient and *in vitro* transduced with a nucleic acid or antigen binding protein of the present invention.

In view of the disclosure herein it will be appreciated that the invention furthermore pertains to the following items:
Item 1: An isolated *antigen binding protein (ABP)* which specifically binds to a Transforming Growth Factor β Receptor 2 (TGFβR2), preferably a mutated TGFβR2, derived antigenic peptide, or a variant thereof, wherein the isolated ABP comprises at least one complementarity determining region (CDR) being a CDR3 having an amino acid sequence with at least 80% sequence identity to, or having no more than three or two, preferably no more than one amino acid substitution(s), deletion(s) or insertion(s) compared to, a sequence selected from SEQ ID Nos: 11, 13, 15, and 17.
Item 2: The isolated ABP of item 1, wherein said ABP is capable of specifically and/or selectively binding to an antigenic peptide derived from a frameshift variant of TGFβR2, preferably wherein the frameshift variant of TGFβR2 is derived from a sequence comprising a frame-shift mutation in the wild type TGFβR2 gene sequence according to SEQ ID No: 1 or 2, more preferably wherein the frame-shift mutation in TGFβR2 is a deletion or an addition of one nucleotide.
Item 3 The isolated ABP of item 2, wherein the frame-shift mutation is a deletion or an addition of one nucleotide in a microsatellite region of the TGFβR2 gene sequence according to SEQ ID No: 1 or 2,
   preferably wherein the microsatellite region is a polyadenine sequence, such as a repeat of adenines located in exon 3 of the wild type TGFβR2 gene sequence according to SEQ ID No: 1 or 2, for example a repeat of adenines within the sequence comprising nucleotide numbers 709 to 718 of the wild type TGFβR2 gene sequence according to SEQ ID No: 1 or 2, and wherein the frame-shift mutation is a deletion or an addition of one adenine.
Item 4 The isolated ABP of item 2 or 3, wherein the frame-shift mutation causes a shift of the open reading frame of the wild type TGFβR2 gene sequence according to SEQ ID No: 1 or 2 due to the deletion or addition of one nucleotide,
   preferably wherein the frameshift variant of TGFβR2 comprises an amino acid at position 128 or 129 according to the numbering in the wild type TGFβR2 amino acid sequence according to SEQ ID No: 5 or 6 that differs from the amino acid at position 128 or 129 of the wild type TGFβR2 amino acid sequence according to SEQ ID No: 5 or 6, more preferably wherein the frameshift variant of TGFβR2 comprises an amino acid sequence according to SEQ ID No: 10.
Item 5 The isolated ABP of any one of items 2 to 4, wherein the frame-shift mutation is a deletion of one nucleotide within the sequence comprising nucleotide numbers 709 to 718 of the wild type TGFβR2 gene sequence according to SEQ ID No: 1 or 2, preferably wherein the deletion of one nucleotide is a deletion of one adenine.
Item 6 The isolated ABP of any one of items 1 to 5, wherein the ABP is an antibody, or an antigen binding derivative or fragment thereof, or, preferably, is a T cell receptor (TCR), or an antigen binding derivative or fragment thereof,
   and/or wherein the ABP binds to a human leucocyte antigen (HLA) presented antigenic peptide, wherein said HLA is preferably a HLA type DR4, preferably wherein the ABP forms a specific complex with the MHC and the antigenic peptide,
   and/or wherein the ABP is humanized, chimerized and/or murinized.
Item 7 The isolated ABP of any one of the preceding items, wherein the ABP specifically and/or selectively binds to an epitope having the amino acid sequence selected from an MHC presented peptide of a frameshift variant of TGFβR2, preferably wherein the peptide comprises a frame-shift mutation in TGFβR2, and most preferably a peptide comprising SEQ ID No: 10.
Item 8 The isolated ABP of any one of the preceding items, wherein the ABP is an α/β-TCR, or an antigen binding derivative or a fragment thereof, or wherein the ABP is a γ/δ-TCR, or an antigen binding derivative or a fragment thereof.
Item 9 The isolated ABP of any one of the preceding items, characterized in that the ABP is of human origin and specifically and/or selectively recognizes a TGFβR2 frameshift antigenic peptide,
   and/or wherein said ABP is capable of inducing an immune response in a subject, optionally wherein the immune response is characterized by an increase in interferon (IFN) γ levels.
Item 10 The isolated ABP of any one of the preceding items, comprising
   a TCR α or γ chain; and/or
   a TCR β or δ chain;
      wherein the TCR α or γ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID Nos: 11 and 15; and/or
      wherein the TCR β or δ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID Nos: 13 and 17.
Item 11 The isolated ABP of any one of the preceding items, comprising a TCR variable chain region having at least 80% sequence identity to, or having no more than 20, preferably no more than 15, more preferably no more than 10, 5, three or two, preferably no more than one amino acid substitution(s), deletion(s) or insertion(s) compared to, an amino acid sequence selected from SEQ ID Nos. 12, 14, 16, and 18.
Item 12 The isolated ABP of any one of the preceding items, comprising a binding fragment of a TCR, and wherein said binding fragment comprises a CDR1 to CDR3, optionally selected from the CDR1 to CDR3 sequences having the amino acid sequences of SEQ ID Nos. 11, 13, 15, and 17; each CDR independently having no more than three or two, preferably no more than one amino acid substitution(s), deletion(s) or insertion(s) compared to these sequences.
Item 13 The isolated ABP of any one of the preceding items, wherein the ABP is a TCR, or an antigen-binding fragment or derivative thereof, comprising at least one TCR α and one TCR β chain sequence,
   wherein said TCR α chain sequence comprises a variable region sequence having the amino acid sequence of SEQ ID No: 12, and
   wherein said TCR β chain sequence comprises a variable region sequence having the amino acid sequence of SEQ ID No: 14; or
   wherein said TCR α chain sequence comprises a variable region sequence having the amino acid sequence of SEQ ID No: 16, and
   wherein said TCR β chain sequence comprises a variable region sequence having the amino acid sequence of SEQ ID No: 18;
   each variable domain sequence having no more than 20, preferably no more than 15, more preferably no more than 10, 5, three or two, preferably no more than one amino acid substitution(s), deletion(s) or insertion(s) compared to these sequences.
Item 14 The isolated ABP of any one of the preceding items, wherein the ABP is a TCR, or an antigen-binding fragment or derivative thereof, further comprising a TCR constant region, preferably a human TCR constant region sequence.
Item 15 An isolated *nucleic acid* encoding for an ABP of any one of items 1 to 14.
Item 16 The isolated nucleic acid of item 15, comprising a nucleic acid sequence having at least 80% sequence identity to, or having no more than 20, preferably no more than 15, more preferably no more than 10, 5, three or two, preferably no more than one nucleic acid substitution(s), deletion(s) or insertion(s) compared to, a nucleic acid sequence encoding for a polypeptide selected from SEQ ID Nos: 12, 14, 16, and 18.
Item 17 A recombinant *vector* comprising a nucleic acid of item 15 or 16.
Item 18 A recombinant *host cell* comprising an ABP of any one of items 1 to 14, or a nucleic acid of item 15 or 16, or a vector of item 17,
   preferably wherein the host cell is a lymphocyte, more preferably a T lymphocyte or T lymphocyte progenitor, even more preferably a CD4 or a CD8 positive T-cell, most preferably a CD4 positive T-cell.
Item 19 A *pharmaceutical composition* comprising
   the ABP of any one of items 1 to 14, or a nucleic acid of item 15 or 16, or a vector of item 17, or the host cell of item 18, and
   a pharmaceutical acceptable carrier, stabilizer and/or excipient;
      preferably wherein the pharmaceutical composition comprises at least two different ABP of any one of items 1 to 14.
Item 20 A compound or composition *for use in medicine*, wherein the compound or composition is selected from an ABP of any one of items 1 to 14, or a nucleic acid of item 15 or 16, or a vector of item 17, or a host cell of item 18, or a pharmaceutical composition of item 19, preferably for use is in the treatment of a proliferative disorder, more preferably cancer, such as a cancer positive for the expression of a TGFβR2 frameshift peptide.
Item 21 The compound or composition for use of item 20, wherein the treatment comprises immune therapy, preferably adoptive autologous or heterologous T-cell therapy.
Item 22 The compound or composition for use of item 21, wherein the T-cell therapy involves the use of a cell comprising the ABP or a nucleic acid of any one of the preceding items.
Item 23 A *method of manufacturing a* TGFβR2 frameshift peptide-specific antigen recognizing construct expressing *cell line*, preferably a TGFβR2(-1) frameshift peptide-specific antigen recognizing construct expressing cell line, comprising
   (a) providing a suitable host cell,
   (b) providing a genetic construct comprising a coding sequence encoding the ABP according to any of items 1 to 14,
   (c) introducing into said suitable host cell said genetic construct, and
   (d) expressing said genetic construct by said suitable host cell.
Item 24 The method of item 28, wherein said ABP is an alpha/beta TCR, gamma/delta TCR, or a TCR variant, such as a single chain TCR (scTCR),
   and/or wherein the suitable host cell is a T-cell,
   preferably a T cell obtained from a patient suffering from a disorder associated with a TGFβR2 frameshift peptide expression, preferably with a TGFβR2(-1) frameshift peptide expression, such as more preferably a cancer, for example colorectal cancer (CRC).

The present invention will now be further described in the following examples with reference to the accompanying figures and sequences, nevertheless, without being limited thereto. For the purposes of the present invention, all references as cited herein are incorporated by reference in their entireties. In the Figures and Sequences:

### BRIEF DESCRIPTION OF THE DRAWINGS AND SEQUENCES

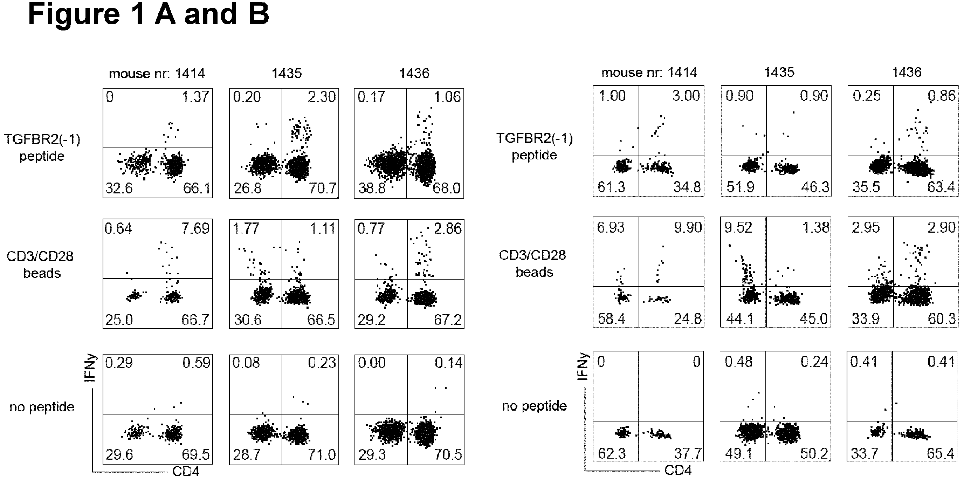

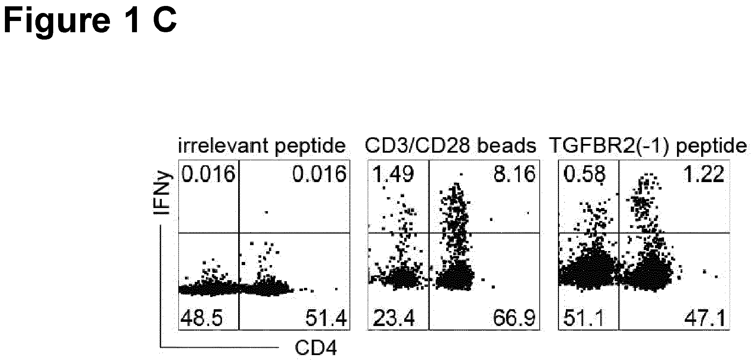

**Figure 1****:** *Generation of TGFβR2(-1)-reactive TCRs in ABabDR4 mice.*
   **A, B.** Intracellular staining (ICS) of PBLs from AbabDR4 mice 7 days after (A) 2^{nd} or (B) 3^{rd} TGFβR2(-1) peptide immunization. Isolated PBLs were stimulated with 10⁻⁶ M TGFβR2(-1) peptide, CD3/CD28 beads were used as positive control and no peptide as negative control. After 2 hrs, Brefeldin A was added to the cultures and ICS was carried out after O/N incubation. Cells are gated on single CD3⁺ lymphocytes. Numbers in gates are population size given in percentage.
   **C.** Sorting of TGFβR2(-1)-reactive IFNy-producing CD4⁺ T cells from spleen of ABabDR4 mouse nr 1414 at day 10 after 3^{rd} peptide immunization. Restimulated splenocytes *in vitro* for 4 hrs with TGFβR2(-1) peptide and CD3/CD28 beads or irrelevant peptide as controls were labelled using mouse IFN-γ Secretion Assay KIT. Labelled cells were sorted by flow cytometry. In total, 6 000 IFNy-producing CD4⁺ TGFβR2(-1)-reactive T cells (1.22%) were collected. Cells are gated on single CD3⁺ lymphocytes. Numbers in gates refer to population size given in percentage.

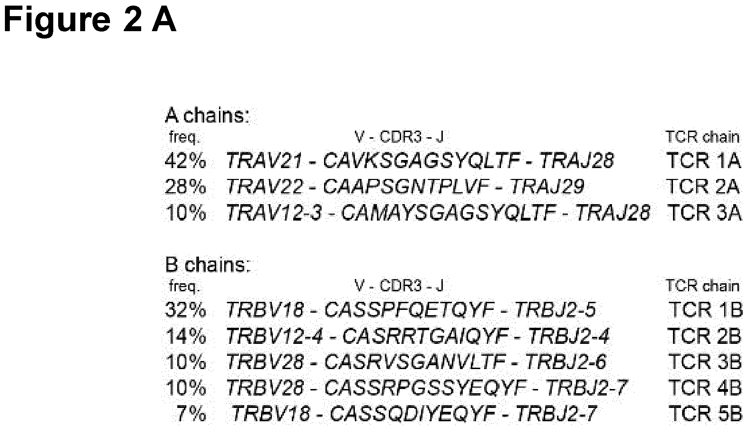

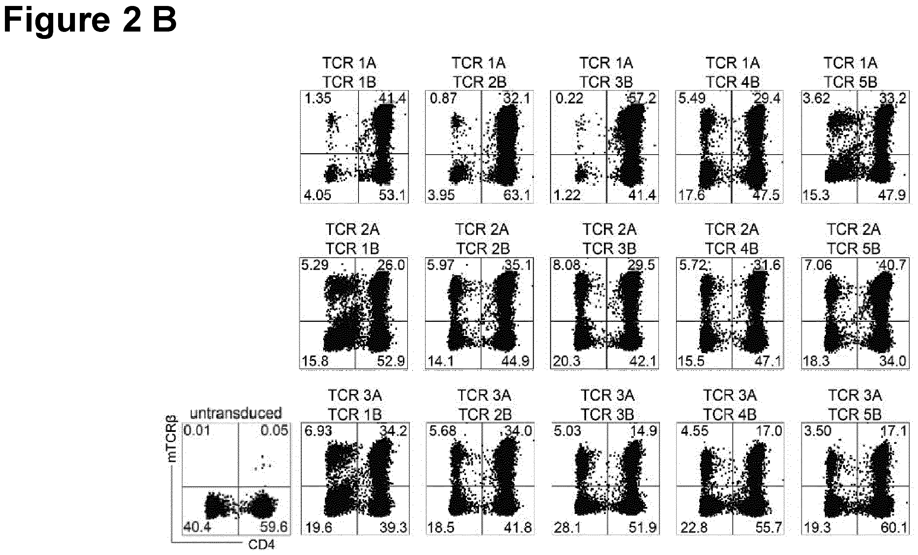

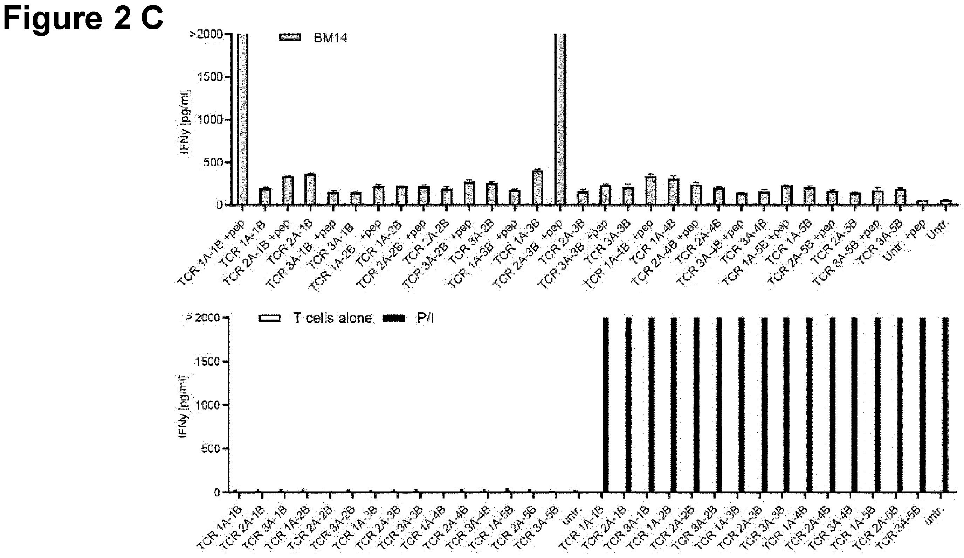

**Figure 2****:** *Analysis of ABabDR4-derived TCR combinations from ABabDR4 mouse nr 1414.*
   **A.** Frequencies of CDR3 regions and V/J gene segments of TCR α- and β- chains. After RACE-PCR from responding CD4⁺ T cells, the amplified PCR product of each chain was separately cloned into TOPO vectors and TOP10 OneShot competent E. coli were transformed. Around 50 clones for each TCRα and TCRβ were analysed and their frequencies are presented in percentages.
   **B.** Combinatorial expression of TCRα and TCRβ genes in human CD4⁺ T cells with given transduction efficiency percentages. Human PBLs-derived CD4⁺ T cells were retrovirally transduced with different TCRα/TCRβ gene combinations and stained with mouse TCRβ constant region (mTCRβ) antibody and human CD4 antibody. Cells are gated on CD3⁺ lymphocytes.
   **C.** Identification of functional pairs TCR 1A 1B and TCR 2A 3B reflected by IFNy secretion measured by ELISA in the supernatant from co-cultured T cells with target cells BM14 (HLA-DR4⁺). Human CD4⁺ PBLs-derived T cells were transduced with 15 ABabDR4-derived TCR combinations. As target, BM14 cells were loaded with TGFβR2(-1)-specific-HLA-DRB1^{∗}04:01-restricted peptide (+pep) at 10⁻⁶ M. As negative control, T cells alone in medium were used and as positive control, T cells with PMA/Ionomycin (P/I). Intra-assay duplicates with standard deviation are displayed. Similar data were obtained with two other cell lines: BSM and K562-DR4 expressing HLA-DR4 (data not shown).

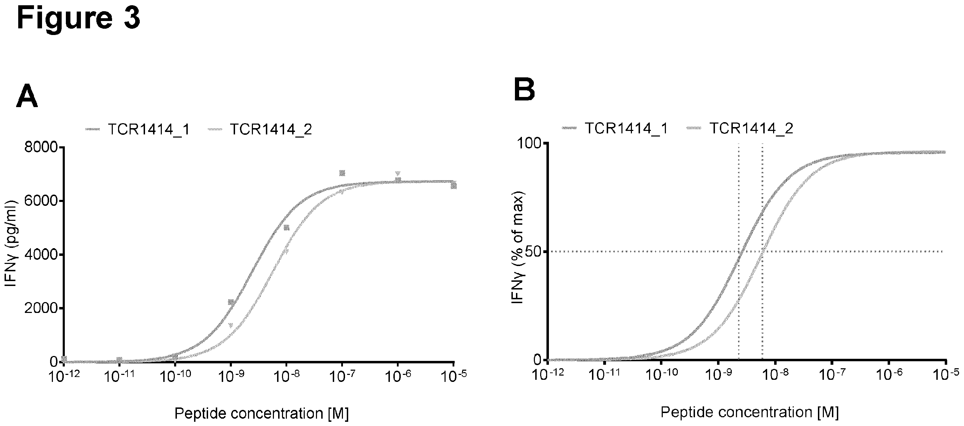

**Figure 3****:** *TCR1414_1 and TCR1414_2 sensitivity to TGFβR2(-1) peptide.*
   **A.** ELISA results reflecting responsiveness of CD4⁺ T cells transduced with TCR1414_1 or TCR1414_2 to varying doses of the TGFβR2(-1) peptide (VALMSAMTTSSSQKN). TCR-transduced CD4⁺ T cells were co-cultured with BSM cells pulsed with decreasing peptide amounts. IFNy was measured in the supernatant after 16 hrs.
   **B.** Normalized data to the maximum IFNy release. Both investigated TCRs show recognition up to 10⁻⁹ M TGFBR2(-1) peptide with IC50 values between 10 and 1 nM for both TCR1414_1 (blue line) and TCR1414_2 (yellow line), as indicated by the red dotted line. The results are representative of 2 independent experiments with 2 different human PBLs donors.

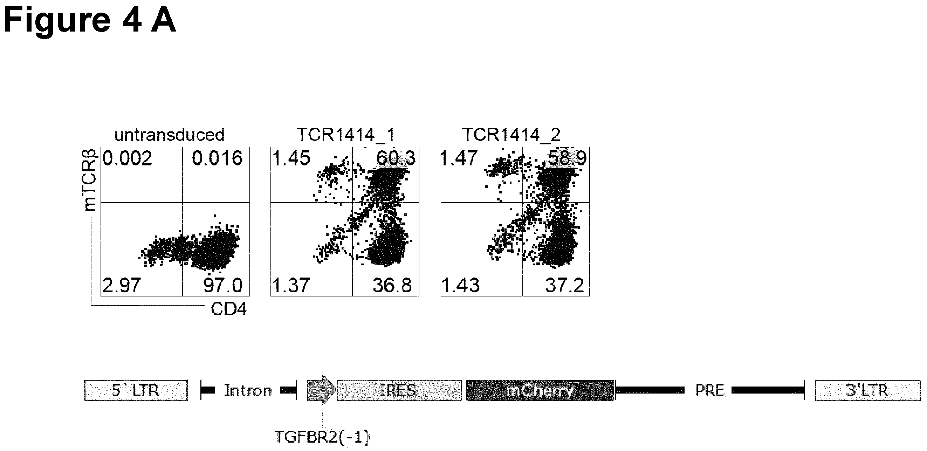

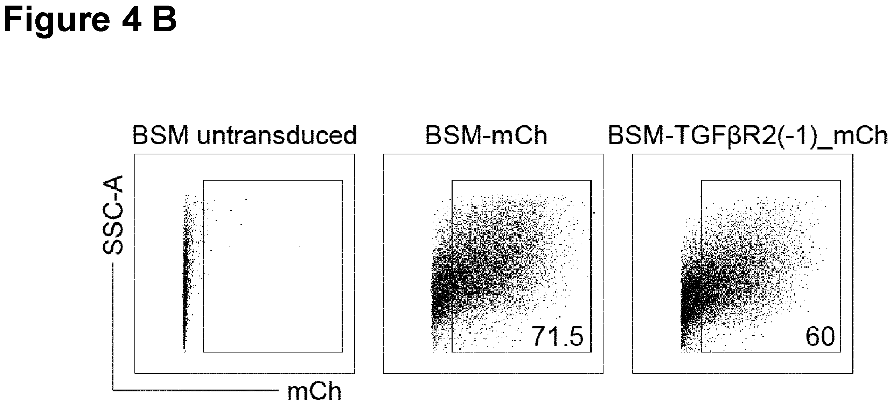

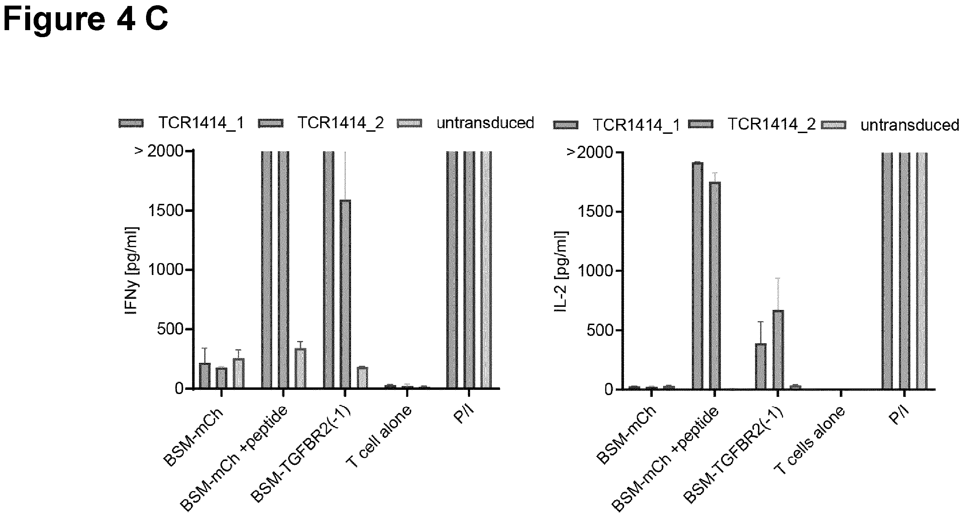

**Figure 4****:** *Recognition of naturally processed TGFβR2(-1) neoantigen by TCR1414_1 and TCR1414_2.*
   **A.** Expression of codon optimized TGFβR2(-1)-reactive TCR1414_1 and TCR1414_2 in human CD4⁺ T cells with given transduction efficiency percentages. TCR1414_1 and TCR1414_2 were retrovirally transduced onto human PBL-derived CD4⁺ T cells (after CD8⁺ T cells depletion) and stained with antibody for mouse TCRβ constant region (mTCRβ) and human CD4. Cells were gated on CD3⁺ lymphocytes. The results are representative of 3 different human PBLs donors.
   **B.** Target cells transduction profile with TGFβR2(-1) minigene. BSM-TGFβR2(-1) was retrovirally transduced with TGFβR2(-1) minigene encoding 47 amino acids and containing HLA-DRB1^{∗}04:01-resctricted epitope of interest coupled to mCherry as reporter. BSM-mCh, was used here as a negative control and transduced with same vector but without the minigene, only mCherry reporter gene. Numbers in gates show transduction frequencies in percentages. **C.** Recognition of naturally processed TGFβR2(-1)-specific HLA-DR4-restricted epitope by TCR-transduced CD4⁺ T cells co-cultured with BSM-TGFβR2(-1) cell line (HLA-DR4⁺) for 16 hrs. Data reflect IFNy and IL-2 production measured by ELISA in supernatant. TGFβR2(-1) peptide at 10⁻⁶ M (+pep.) or PMA/Ionomycin (P/I) were added as indicated. Results are representative of 3 (for IFNy ELISA) and 2 (for IL-2 ELISA) independent experiments with different PBLs donors.

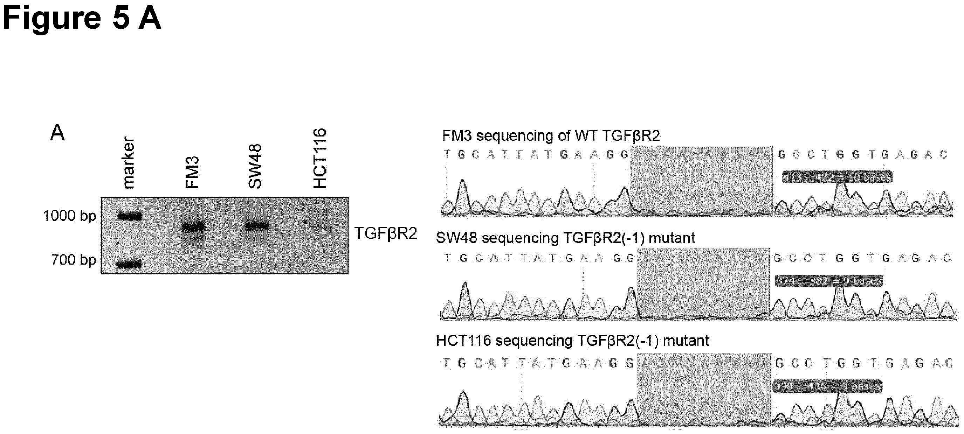

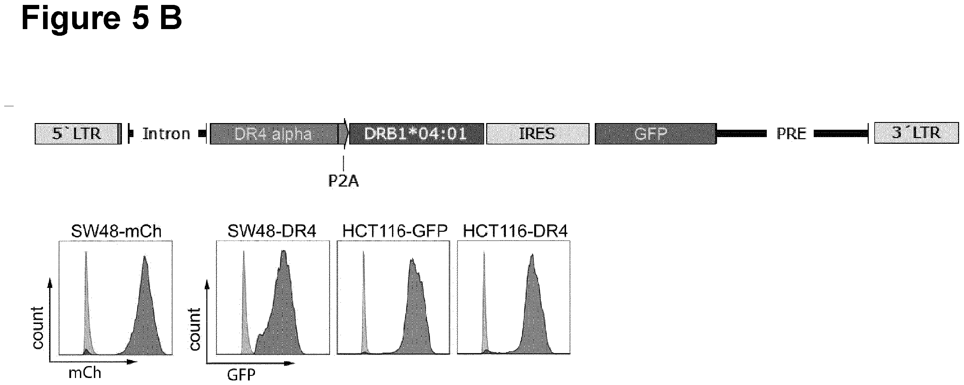

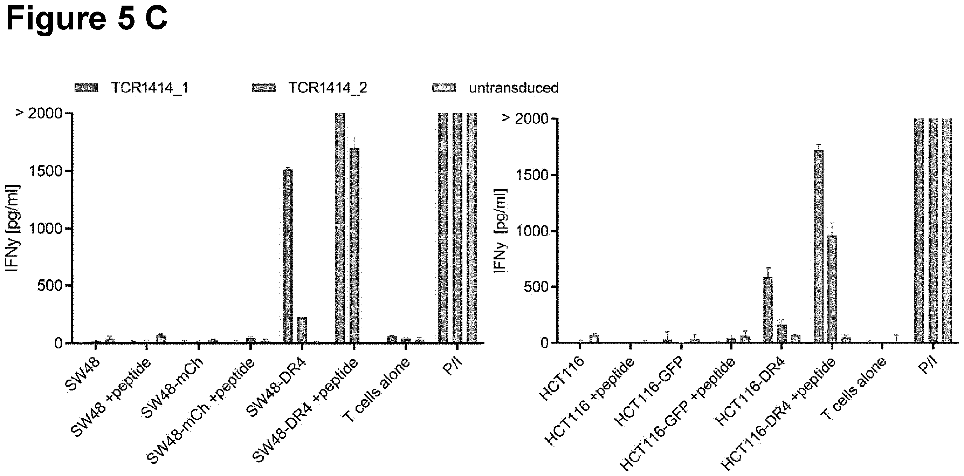

**Figure 5****:** *Recognition of naturally expressed TGFβR2(-1) antigen in colorectal cancer (CRC) cell lines by TCR1414_1 and TCR14141_2.*
   **A.** TGFβR2(-1) mutation detection in CRC cell lines SW48 and HCT116. Electrophoresis of amplified PCR products from cDNA shows the TGFβR2 region of interest containing the frameshift mutation. Primers for PCR were designed to cover the two splicing variants of the TGFβR2 gene. Sanger sequencing results show one adenine deletion (9A) in the 10-adenine microsatellite of TGFβR2 gene in both CRC cell lines. As control, a FM3 melanoma cell line containing the wild type (WT) TGFβR2 DNA sequence was used. Data represent one out of 6 clones analysed for each CRC cell line.
   **B.** CRC cell lines SW48 and HCT116 (both HLA-DR^{∗}04:01⁻) were transduced with HLA-DRA/HLA-DRB1^{∗}0401 construct, as indicated, or mock controls (mCh or GFP). Dark grey represents transduced cells and light grey, untransduced, parental cell line. Expression of reporter genes GFP or mCh was measured by flow cytometry.
   **C.** Recognition of naturally expressed TGFβR2(-1) neoantigen in SW48 and HCT116 cell lines by TCR1414_1 and TCR14141_2. TCR-transduced human CD4⁺ T cells were co-cultured with CRC cell lines SW48-DR4 and HCT116-DR4 that endogenously express TGFβR2(-1) frameshift mutation. As positive controls, PMA and Ionomycin (P/I) were used, whereas for negative control, T cells alone were used. After 24h of incubation, IFNy was measured in the supernatant by ELISA. The results are representative of 4 independent experiments performed with 3 different PBLs donors.

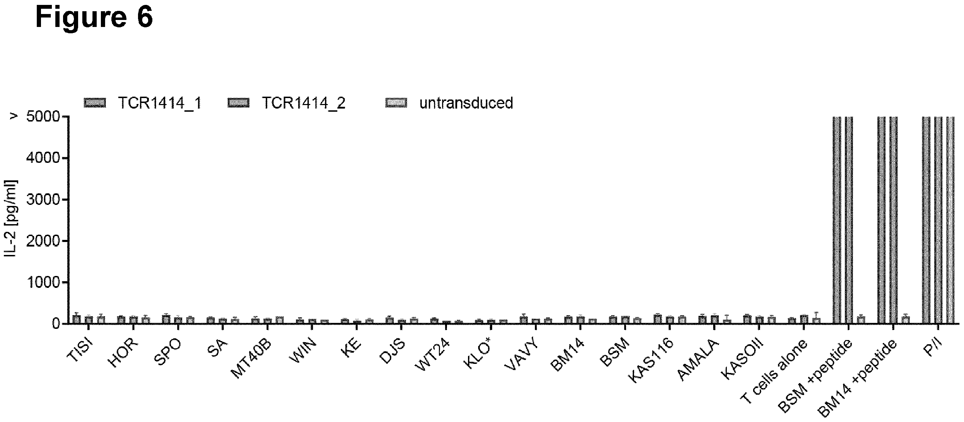

**Figure 6****:** *Alloreactivity test of ABabDR4-derived TCRs.*
   TCR1414_1 and TCR1414_2 transduced human CD4⁺ T cells were co-cultured with a panel of B-LCLs expressing different HLA allotypes, including both MHC I and II molecules (full HLAs profile of each LCL in Table 2). As positive control, PMA/Ionomycin (P/I) and BSM, BM14 (both HLA-DR^{∗}04:01⁺) loaded with 10⁻⁶ M TGFβR2(-1) peptide were used, whereas for negative control T cells alone were used. After 24h of incubation, IL-2 was measured in the supernatant by ELISA. The results are representative of 3 independent experiments performed with T cells from 3 different PBLs donors. For all experiments, all cell lines were used simultaneously except for ^{∗}KLO cell line.

**SEQ ID NO. 1:** cDNA gene sequence of TGFβR2 Isoform 1 (4530 bp).
   See Sequence listing.
**SEQ ID NO. 2:** cDNA gene sequence of TGFβR2 Isoform 2 (4605 bp).
   See Sequence listing.
**SEQ ID NO. 3:** Gene sequence of TGFβR2(-1) Isoform 1: 
ACTCGCGCGCACGGAGCGACGACACCCCCGCGCGTGCACCCGCTCGGGACAGGAGCCGGACTCCTGTG
CAGCTTCCCTCGGCCGCCGGGGGCCTCCCCGCGCCTCGCCGGCCTCCAGGCCCCCTCCTGGCTGGCGA
GCGGGCGCCACATCTGGCCCGCACATCTGCGCTGCCGGCCCGGCGCGGGGTCCGGAGAGGGCGCGGCG
CGGAGGCGCAGCCAGGGGTCCGGGAAGGCGCCGTCCGCTGCGCTGGGGGCTCGGTCTATGACGAGCAG
CGGGGTCTGCCATGGGTCGGGGGCTGCTCAGGGGCCTGTGGCCGCTGCACATCGTCCTGTGGACGCGT
ATCGCCAGCACGATCCCACCGCACGTTCAGAAGTCGGTTAATAACGACATGATAGTCACTGACAACAA
CGGTGCAGTCAAGTTTCCACAACTGTGTAAATTTTGTGATGTGAGATTTTCCACCTGTGACAACCAGA
AATCCTGCATGAGCAACTGCAGCATCACCTCCATCTGTGAGAAGCCACAGGAAGTCTGTGTGGCTGTA
TGGAGAAAGAATGACGAGAACATAACACTAGAGACAGTTTGCCATGACCCCAAGCTCCCCTACCATGA
CTTTATTCTGGAAGATGCTGCTTCTCCAAAGTGCATTATGAAGGAAAAAAAAAGCCTGGTGAGACTTT
CTTCATGTGTTCCTGTAGCTCTGATGAGTGCAATGACAACATCATCTTCTCAGAAGAATATAACACCA
GCAATCCTGACTTGTTGC
**SEQ ID NO. 4:** Gene sequence of TGFβR2(-1) Isoform 2: 
ACTCGCGCGCACGGAGCGACGACACCCCCGCGCGTGCACCCGCTCGGGACAGGAGCCGGACTCCTGTG
CAGCTTCCCTCGGCCGCCGGGGGCCTCCCCGCGCCTCGCCGGCCTCCAGGCCCCCTCCTGGCTGGCGA
GCGGGCGCCACATCTGGCCCGCACATCTGCGCTGCCGGCCCGGCGCGGGGTCCGGAGAGGGCGCGGCG
CGGAGGCGCAGCCAGGGGTCCGGGAAGGCGCCGTCCGCTGCGCTGGGGGCTCGGTCTATGACGAGCAG
CGGGGTCTGCCATGGGTCGGGGGCTGCTCAGGGGCCTGTGGCCGCTGCACATCGTCCTGTGGACGCGT
ATCGCCAGCACGATCCCACCGCACGTTCAGAAGTCGGATGTGGAAATGGAGGCCCAGAAAGATGAAAT
CATCTGCCCCAGCTGTAATAGGACTGCCCATCCACTGAGACATATTAATAACGACATGATAGTCACTG
ACAACAACGGTGCAGTCAAGTTTCCACAACTGTGTAAATTTTGTGATGTGAGATTTTCCACCTGTGAC
AACCAGAAATCCTGCATGAGCAACTGCAGCATCACCTCCATCTGTGAGAAGCCACAGGAAGTCTGTGT
GGCTGTATGGAGAAAGAATGACGAGAACATAACACTAGAGACAGTTTGCCATGACCCCAAGCTCCCCT
ACCATGACTTTATTCTGGAAGATGCTGCTTCTCCAAAGTGCATTATGAAGGAAAAAAAAAGCCTGGTG
AGACTTTCTTCATGTGTTCCTGTAGCTCTGATGAGTGCAATGACAACATCATCTTCTCAGAAGAATAT
AACACCAGCAATCCTGACTTGTTGC
**SEQ ID NO. 5:** Amino acid sequence of human TGFβR2 Isoform 1 (567 amino acids long):
**SEQ ID NO. 6:** Amino acid sequence of human TGFβR2 Isoform 2 (592 amino acids long): 
MGRGLLRGLWPLHIVLWTRIASTIPPHVQKSDVEMEAQKDEIICPSCNRTAHPLRHINNDMIVTDNNG
AVKFPQLCKFCDVRFSTCDNQKSCMSNCSITSICEKPQEVCVAVWRKNDENITLETVCHDPKLPYHDF
ILEDAASPKCIMKEKKKPGETFFMCSCSSDECNDNIIFSEEYNTSNPDLLLVIFQVTGISLLPPLGVA
ISVIIIFYCYRVNRQQKLSSTWETGKTRKLMEFSEHCAIILEDDRSDISSTCANNINHNTELLPIELD
TLVGKGRFAEVYKAKLKQNTSEQFETVAVKIFPYEEYASWKTEKDIFSDINLKHENILQFLTAEERKT
ELGKQYWLITAFHAKGNLQEYLTRHVISWEDLRKLGSSLARGIAHLHSDHTPCGRPKMPIVHRDLKSS
NILVKNDLTCCLCDFGLSLRLDPTLSVDDLANSGQVGTARYMAPEVLESRMNLENVESFKQTDVYSMA
LVLWEMTSRCNAVGEVKDYEPPFGSKVREHPCVESMKDNVLRDRGRPEIPSFWLNHQGIQMVCETLTE
CWDHDPEARLTAQCVAERFSELEHLDRLSGRSCSEEKIPEDGSLNTTK
**SEQ ID NO. 7:** Amino acid sequence of human TGFβR2 Isoform 1 (112 amino acids long) comprising the TGFβR2(-1) frameshift mutation (frameshift sequence underlined): 
MGRGLLRGLWPLHIVLWTRIASTIPPHVQKSVNNDMIVTDNNGAVKFPQLCKFCDVRFSTCDNQKSCM
SNCSITSICEKPQEVCVAVWRKNDENITLETVCHDPKLPYHDFILEDAASPKCIMKEKKSLVRLSSCV
PVALMSAMTTSSSQKNITPAILTCC
**SEQ ID NO. 8:** Amino acid sequence of human TGFβR2 Isoform 2 (127 amino acids long) comprising the TGFβR2(-1) frameshift mutation (frameshift sequence underlined): 
MGRGLLRGLWPLHIVLWTRIASTIPPHVQKSDVEMEAQKDEIICPSCNRTAHPLRHINNDMIVTDNNG
AVKFPQLCKFCDVRFSTCDNQKSCMSNCSITSICEKPQEVCVAVWRKNDENITLETVCHDPKLPYHDF
ILEDAASPKCIMKEKKSLVRLSSCVPVALMSAMTTSSSQKNITPAILTCC
**SEQ ID NO. 9:** TGFβR2(-1) frameshift peptide:
   SLVRLSSCVPVALMSAMTTSSSQKNITPAILTCC
**SEQ ID NO. 10:** Short version of the TGFβR2(-1) frameshift peptide (DR4-restricted epitope chosen for immunization):
   VALMSAMTTSSSQKN

**Table 1: Isolated TCR amino acid sequences of the invention**

| **TCR Designation** | **TCR Chain** | **TCR Domain** | **Sequence** | **SEQ ID NO:** |
|---|---|---|---|---|
| TCR1414_1 | alpha | CDRA3 | TRAV21 - CAVKSGAGSYQLTF - TRAJ28 | **11** |
| TCR1414_1 | alpha | Variable Domain A | METLLGLLILWLQLQWVSSKQEVTQIPAALSVPEGENL VLNCSFTDSAIYNLQWFRQDPGKGLTSLLLIQSSQREQ TSGRLNASLDKSSGRSTLYIAASQPGDSATYLCAVKSG AGSYQLTFGKGTKLSVIP | **12** |
| TCR1414_1 | beta | CDRB3 | TRBV18 - CASSPFQETQYF - TRBJ2-5 | **13** |
| TCR1414_1 | beta | Variable Domain B | MDTRVLCCAVICLLGAGLSNAGVMQNPRHLVRRRGQEA RLRCSPMKGHSHVYWYRQLPEEGLKFMVYLQKENIIDE SGMPKERFSAEFPKEGPSILRIQQVVRGDSAAYFCASS PFQETQYFGPGTRLLVL | **14** |
| TCR1414_2 | alpha | CDRA3 | TRAV22 - CAAPSGNTPLVF - TRAJ29 | **15** |
| TCR1414_2 | alpha | Variable Domain A | MKRILGALLGLLSAQVCCVRGIQVEQSPPDLILQEGAN STLRCNFSDSVNNLQWFHQNPWGQLINLFYIPSGTKQN GRLSATTVATERYSLLYISSSQTTDSGVYFCAAPSGNT PLVFGKGTRLSVIA | **16** |
| TCR1414_2 | beta | CDRB3 | TRBV28 - CASRVSGANVLTF - TRBJ2-6 | **17** |
| TCR1414_2 | beta | Variable Domain B | MGIRLLCRVAFCFLAVGLVDVKVTQSSRYLVKRTGEKV FLECVQDMDHENMFWYRQDPGLGLRLIYFSYDVKMKEK GDIPEGYSVSREKKERFSLILESASTNQTSMYLCASRV SGANVLTFGAGSRLTVL | **18** |

**Table 2: Isolated TCR nucleotide sequences of the invention**

| **TCR Designation** | **TCR Chain** | **TCR Domain** | **Sequence** | **SEQ ID NO:** |
|---|---|---|---|---|
| TCR1414_1 | alpha | CDRA3 | TGTGCCGTGAAAAGCGGAGCCGGCAGCTACCAGCTGACAT TT | **19** |
| TCR1414_1 | alpha | Variable Domain A | ATGGAAACACTGCTGGGCCTGCTGATCCTGTGGCTGCAAC TGCAATGGGTGTCCAGCAAGCAAGAAGTGACCCAGATTCC TGCCGCTCTGTCTGTGCCAGAGGGCGAGAATCTGGTCCTG AACTGCAGCTTCACCGACAGCGCCATCTACAACCTGCAGT GGTTCAGACAGGACCCCGGCAAGGGACTGACAAGCCTGCT GCTGATTCAGAGCAGCCAGAGAGAGCAGACCAGCGGCAGA CTGAACGCCAGCCTGGACAAGTCTAGCGGCAGAAGCACCC TGTATATCGCCGCTTCTCAGCCTGGCGACTCTGCCACATA TCTGTGTGCCGTGAAAAGCGGAGCCGGCAGCTACCAGCTG ACATTTGGCAAGGGCACCAAGCTGAGCGTGATCCCC | **20** |
| TCR1414_1 | beta | CDRB3 | TGTGCTAGCAGCCCCTTCCAAGAGACACAGTACTTC | **21** |
| TCR1414_1 | beta | Variable Domain B | ATGGATACAAGAGTGCTGTGTTGCGCCGTGATCTGCCTGC TTGGAGCCGGACTTTCTAATGCCGGCGTGATGCAGAACCC CAGACACCTCGTTCGGAGAAGAGGCCAAGAGGCCAGACTG AGATGCAGCCCTATGAAGGGCCACAGCCACGTGTACTGGT ACAGACAGCTGCCTGAAGAGGGCCTGAAGTTCATGGTGTA CCTGCAGAAAGAGAACATCATCGACGAGAGCGGCATGCCC AAAGAGCGGTTCTCTGCCGAGTTTCCCAAAGAGGGCCCCA GCATCCTGAGAATCCAGCAGGTTGTGCGGGGAGATAGCGC CGCCTACTTTTGTGCTAGCAGCCCCTTCCAAGAGACACAG TACTTCGGCCCTGGCACCAGACTGCTGGTGCTG | **22** |
| TCR1414_2 | alpha | CDRA3 | TGTGCCGCTCCTAGCGGCAATACCCCTCTGGTGTTT | **23** |
| TCR1414_2 | alpha | Variable Domain A | ATGAAGAGAATCCTGGGAGCCCTGCTGGGACTGCTGAGTG CTCAAGTGTGCTGTGTGCGGGGCATCCAGGTGGAACAGTC TCCACCAGATCTGATCCTGCAAGAGGGCGCCAACAGCACC CTGAGATGCAACTTCAGCGACAGCGTGAACAACCTGCAGT GGTTCCACCAGAATCCTTGGGGCCAGCTGATCAACCTGTT CTACATCCCCAGCGGCACCAAGCAGAACGGCAGACTGTCT GCAACCACCGTGGCCACCGAGAGATACTCCCTGCTGTACA TCTCCAGCAGCCAGACCACAGACTCCGGCGTGTACTTTTG TGCCGCTCCTAGCGGCAATACCCCTCTGGTGTTTGGCAAG GGCACAAGACTGAGCGTGATCGCCA | **24** |
| TCR1414_2 | beta | CDRB3 | TGTGCCAGCAGAGTGTCCGGCGCCAATGTGCTGACATTT | **25** |
| TCR1414_2 | beta | Variable Domain B | ATGGGAATTAGACTGCTGTGCAGAGTGGCCTTCTGCTTCC TGGCTGTTGGCCTGGTGGACGTGAAAGTGACCCAGAGCAG CAGATACCTGGTCAAGAGAACCGGCGAGAAGGTGTTCCTG GAATGCGTGCAGGACATGGACCACGAGAATATGTTCTGGT ACAGACAGGACCCCGGCCTGGGCCTGAGACTGATCTACTT CAGCTACGACGTGAAGATGAAGGAAAAGGGCGACATCCCC GAGGGCTACAGCGTGTCCAGAGAGAAGAAAGAGCGGTTCA GCCTGATCCTGGAAAGCGCCAGCACCAACCAGACCAGCAT GTACCTGTGTGCCAGCAGAGTGTCCGGCGCCAATGTGCTG ACATTTGGAGCCGGCAGCAGACTGACCGTGCTG | **26** |

### EXAMPLES

### Example 1: Materials and Methods

### 1.1 Cell lines and cell culture

The human CRC cell line HCT116 (Brattain *et al.*, 1981) and SW48 (Leibovltz *et al.*, 1976) were obtained from Prof G. Willimsky and SW48 from Prof. U. Stein, Berlin, respectively. Cell line SW48-DR4, HCT116-DR4 were generated by retroviral transduction with HLA-DRA and HLA-DRB1^{∗}0401 (HLA-DR4) cDNAs fused by the self-cleaving element P2A. The HLA-DR4 DNA construct was introduced into MP71_IRES_GFP vector using restriction sites NotI and SalI. For generation of BSM-TGFβR2(-1) cell line (HLA-DR4⁺), the TGFβR2(-1) minigene was first synthetized (GeneArt). Next, using restriction sites PmlI introduced via PCR, the TGFβR2(-1) minigene was cloned into the MP71_mCh vector (Engels *et al.*, 2003). The BSM-TGFβR2(-1) cell line (HLA-DR4⁺) was generated by retroviral transduction of the TGFβR2(-1)_MP71_mCh vector (Engels *et al.,* 2003). The human melanoma cell line FM3 was provided by the European Searchable Tumor Cell Bank and Database (ESTDAB). All cell lines, including the LCL panel (EBV-transformed lymphoblastoid B cell lines), were maintained in RPMI-1640 medium supplemented with 10% heat-inactivated fetal calf serum (FCS; PAN Biotech), 1× antibiotic-antimycotic, 1 mM sodium pyruvate and 100 µM non-essential amino acids (all reagents were purchased from Life Technologies). The CRC cell lines' medium was additionally supplemented with gentamicin (Life Technologies). The packaging cell line 293GP-GLV (GALV cells) (Ghani *et al.*, 2009) producing retroviral particles was cultured in Dulbecco's modified Eagles medium (DMEM) supplemented with 10% FCS and 1× antibiotic-antimycotic. The primary human PBL-derived T cells were kept in RPMI-1640 medium supplemented with 1 mM HEPES, 10% heat-inactivated fetal calf serum, 1× antibiotic-antimycotic, 1 mM sodium pyruvate and optionally with 300 U/ml or 30 U/ml IL 2.

### 1.2 Mouse ABabDR4 strain

TCRs were generated in transgenic ABabDR4 mice expressing a diverse human TCRαβ repertoire restricted to human MHC class II molecule HLA-DRA/DRB1^{∗}0401 (Li *et al.*, 2010; Chen *et al.*, 2017). All animal experiments were approved and conducted according to national guidelines (Landesamt für Gesundheit und Soziales, Berlin, Germany). All mice were kept at the animal facility of the Max-Delbrück Center for Molecular Medicine under SPF (specific-pathogen-free) conditions.

### 1.3 Immunization of ABabDR4 mice

ABabDR4 mice were immunized at an age of 8 to 12 weeks using peptide immunization with TGFβR2(-1)-specific HLA-DR4-restricted peptide (VALMSAMTTSSSQKN, GenScript) [SEQ ID NO. 10]. The 80 µg of peptide dissolved in PBS were added to 50 µg of oligonucleotide CpG (CpG 1826, MOLBIOL) and 100 µl of incomplete Freund's adjuvant (IFA, Sigma-Aldrich). A volume of 200 µl emulsion (peptide and CpG dissolved in 100 µl PBS plus 100 µl IFA) was injected subcutaneously into mice. If necessary, immunizations were repeated at 4-week intervals.

### 1.4 ICS (Intracellular Staining)

One week after the last immunization, the peripheral blood of immunized ABabDR4 mice was analysed for the presence of responding CD4⁺ T cells. The cell fraction after lysing red blood cells with ACK buffer (150 mM NH₄Cl, imM KHCO₃, 100 nM Na₂EDTA) was stimulated with TGFβR2(-1)-specific HLA-DR4-restricted peptide at 1 µM, no peptide as negative control or 20 µl containing 8^{∗}10⁵ CD3/CD28 Dynabeads T activator (Invitrogen) as positive control. After 2 hours, protein transport inhibitor Brefeldin A (BD Golgie Plug) was added and intracellular IFNy staining was performed after 13 hours of incubation in 37°C. After Fc blocking (antimouse CD16/32, BioLegend), cells were fixed using BD Cytofix/Cytoperm KIT (BD Bioscience) and stained with 50 µg/ml of anti-CD3, -CD4 and -IFNy antibodies (BioLegend) for 30 min at 4°C, then analysed by flow cytometry.

### 1.5 IFNy secretion assay

Ten days after the last immunization, mice were sacrificed and splenocytes were isolated. Next, after 4 hours of restimulation with TGFβR2(-1)-specific HLA-DR4-restricted peptide at 1 µM, cells producing IFNy were stained using IFN-y Secretion Assay KIT (Miltenyi Biotec). Positive CD4⁺ T cells producing IFNy were collected by FACS into lysate buffer RTL Plus (RNeasy Micro Kit, QIAGEN) for TCRs isolation.

### 1.6 TCR isolation

From positive CD4⁺ T cells sorted by FACS during IFN-y secretion assay (Miltenyi Biotec), RNA was isolated (RNeasy Micro Kit, QIAGEN). This was followed by synthesis of cDNA and 5' RACE PCR (SMARTer RACE cDNA Amplification Kit, Clontech) with 0.5 µM primers specific for the constant region of either TCRα (5'-CGGCCACTTTCAGGAGGAGGATTCGGAAC-3') [SEQ ID NO. 27] or TCRβ (5'-CCGTAGAACTGGACTTGACAGCGGAAGTGG-3') [SEQ ID NO. 28] chain. Each reaction was performed in volumes of 50 µl with 0.5 µl Phusion High-Fidelity DNA polymerase (New England Biolabs), 10 mM dNTPs, 1× Universal Primer Mix (10×, UPM, Clontech). Thermocycler conditions were as follows: 2 min at 98°C, 5 cycles of 98°C for 30s and 72°C for 45s, 5 cycles of 98°C for 30s, 68°C for 30s and 72°C for 45s, 25 cycles of 98°C for 20s, 68°C for 20s and 72°C for 45s, final elongation at 72°C for 5 min. The RACE PCR products were purified from an electrophoresis gel and cloned into TOPO vectors (Zero Blunt TOPO PCR cloning Kit, Invitrogen) to transform TOP10 OneShot competent E. coli (Invitrogen). Depending on clonality, around 50 clones per TCR chain were sequenced and further analyzed.

### 1.7 Generation of TCR transgene cassettes in MP71 vector

Isolated and amplified TCRα and TCRβ chains contain human constant regions. To reduce potential cross pairing with endogenous TCRs, we exchanged the human constant regions for the mouse counterparts (Cohen *et al.*, 2006). To this purpose, overlapping extension PCR (OE-PCR) was performed from two PCR products. The 1^{st} PCR murine constant region was amplified from an already murinized TCR-3600 vector (Poncette *et al.*, 2018). Primers alpha_ms_fwd and huTCR3600_rvs were used to amplify murine alpha constant region. Primers beta_ms_fwd and TCR3600_beta_EcoR1_rvs were used to amplify murine constant beta region. The 2^{nd} PCR included TCRα/β variable gene amplification using primers specific for each TRAV/TRBV with V(D)J_alpha_rvs and VDJ_beta_rvs, respectively (all primers listed below). The 3^{rd} OE-PCR was the final one to combine variable regions of each TCRα/β with a constant murine region. Such full length single TCRα and TCRβ transgenes were cloned using NotI and EcoRI restriction sites into MP71 vector for combinatorial expression of different TCRα + TCRβ and to identify functional TCRαβ pairs (Engels *et al.*, 2003). Functional TCRα + TCRβ pairs were synthesized with *Homo Sapiens* codon optimization (GeneArt) for better expression in human PBLs. Chains TCRβ with TCRα were linked with the porcine teschovirus-i-derived self-cleaving peptide P2A (Leisegang *et al.*, 2008). Constant region remained murinized. The synthesized expression cassettes encoded two TCRs: TCR1414_1 or TCR1414_2.

| | | SEQ ID NO. |
|---|---|---|
| alpha_ms_fwd | 5'-ATATCCAGAACCCCGAGCCTGCCGTGTACC-3' | 29 |
| huTCR3600_rvs | 5'-CAGGAATTCTCATCAGCTGGACCAC-3' | 30 |
| beta_ms_fwd | 5'-GGATCTGAGAAACGTGACCCCCCCCAAGGT-3' | 31 |
| TCR3600_beta_ EcoR1_rvs | 5'-ACTGAATTCTCAGCTGTTCTTCTTCTTGACCATGG-3' | 32 |
| V(D)J_alpha_rvs | 5'-AGCTGGTACACGGCAGGCTCGGGGTTCTG-3' | 33 |
| TRAV21_Not1 | 5'-ATTGCGGCCGCCATGGAGACCCTCTTGGGCC-3 | 34 |
| TRAV22 Not1 | 5'-ATTGCGGCCGCCATGAAGAGGATATTGGGAGC-3' | 35 |
| TRAV12-3_Not1 | 5'-GCATTGCGGCCGCCATGATGAAATCCTTGAGAGTTTTAC-3' | 36 |
| VDJ_beta_rvs | 5'-ACCTTGGGGGGGGTCACGTTTCTCAGATCC-3' | 37 |
| TRBV28_Not1 | 5'-ATTGCGGCCGCCATGGGAATCAGGCTCCTCTG-3' | 38 |
| TRBV12-4-Not1 | 5'-ATTGCGGCCGCCATGGGCTCCTGGACCC-3' | 39 |
| TRBV18_Not1 | 5'-TATGCGGCCGCCATGGACACCAGAGTACTCTGC-3' | 40 |

### 1.8 Transfection of packaging cell lines GALV

To produce amphotropic TCR-bearing retroviral particles, 293GP-GLV (GALV) cells were used. Virus supernatant was produced by transfecting GALV cells (Ghani *et al.,* 2009) with the retroviral vector MP71 (Engels *et al.,* 2003) containing TCR expression cassette with TCR1414_1 or TCR14141_2. For transduction of HCT116 and SW48 cell lines, GALV supernatants were prepared with vector MP71 containing HLA-DR4_GFP, GFP, TGFβR2(-1)_mCh or mCh. GALV cells seeded at 7×10⁵ per well in 6-well plates were transfected with 3 µg of plasmid DNA using either lipofectamine 2000 reagent (Thermo Fisher Scientific) or 18 µg of DNA when calcium chloride (Sigma Aldrich) method was used (Soneoka *et al.,* 1995). 48h and 72h after transfection, 3 ml of virus supernatant were harvested, filtrated (0.45 µm pore size) and used directly for transduction or stored at -80°C.

### 1.9 Transduction of primary T cells

Human primary T cells were transduced with TCRs after CD8⁺ cell depletion from PBLs using MACS anti-CD8 MicroBeads human and LD separation column (Miltenyi, cat. nr 130-045-201). One million CD8 depleted PBLs per well were seeded in 24-well plates (non-tissue culture, Thermo), previously coated with 5 µg/ml anti-CD3 (OKT3, BD Pharmingen) and 1 µg/ml anti-CD28 antibodies (CD28.2, BD Pharmingen) in 1 ml T cell medium supplemented with 200 U/ml IL-2. After 2 days, activated T cells were transduced with 1 ml of TCR1414_1-GALV or TCR1414_2-GALV supernatant supplemented with 4 µg/ml protamine sulfate (Sigma-Aldrich) and spinoculated for 90 min at 32°C and 800g. A second transduction was performed the next day on RetroNectin-coated 6-well plates (25 µg/ml in PBS), pre-coated with virus particles for 2 h at 32°C and 2000g. PBLs were transferred into virus-coated 6-well-plate and spinoculated for 30 min at 800g. Transduced PBLs were expanded for 7 days in T cell medium supplemented with 300 U/ml IL-2. For the next 3-5 days, they were transferred into low IL-2 medium (30 U/ml). After this resting phase, transduced PBLs were ready to be used for co-culture assays or were frozen and stored in liquid nitrogen. For transduction of single TCR α or β chain combinations, virus supernatants were mixed 1:1 in 1 ml volume.

### 1.10 Co-culture experiments

Co-culture of human T cells transduced with different non-codon optimized TCRα + TCRβ combinations were incubated with BM14 cells (HLA-DR4⁺) as target cells at ratio 1:5, where 1×10⁴ transduced CD4⁺ T cells were seeded with 5×10⁴ BM14 target cells in round bottom 96-well plates for 16-18 hrs. Transduction efficiencies were in a range of ~30%. Co-culture of human T cells transduced with codon optimized TCR1414_1 or TCR1414_2 was incubated with target cells (BSM, BM14, SW48, HCT116 or panel of LCLs) at a ratio 1:1, where 1×10⁴ transduced CD4⁺ T cells were seeded with 1×10⁴ target cells in round bottom 96-well plates for 20-24h. Transduction efficiencies were in a range of ~60%. As positive control, 50 ng/ml of Phorbol 12-myristate 13-acetate (PMA) and 5 µg/ml Ionomycin were added to T cells alone. Negative controls were transduced T cells alone seeded in medium. TGFβR2(-1) peptide (VALMSAMTTSSSQKN, GenScript) [SEQ ID NO. 10] was added at 10⁻⁶ M unless indicated differently. After incubation, supernatant was collected and IL-2 or IFNy concentrations were measured by enzyme-linked immunosorbent assay (ELISA; BD OptEIA).

### 1.11 ELISA

ELISA microwells were coated with 50 µl/well of capture antibody appropriately diluted in coating buffer and incubated overnight at 4°C. Next day, wells were washed 3 times with wash buffer. After that, plates were blocked with 100 µl/well assay diluent (10% FCS in PBS) and incubated at room temperature (RT) for 1 hour. Next, wells were washed 3 times with Wash Buffer. 50 µl of prepared standards, controls and samples were distributed into appropriate wells. If needed, samples were diluted in assay diluent. Plates were sealed and incubated for at least 2 hours at RT. Then, wells were washed as before, but with 5 total washes. A volume of 50 µl of working detector (detection antibody + Streptavidin-Horseradish peroxidase (SAv-HRP) reagent) was added to each well and plates were incubated for 1 hour at RT. After the last extensive wash (8 times), 50 µl of Substrate Solution were added to each well and incubated for about 5 min at RT in the dark. Next 25 µl of stop solution were added to each well. Absorbance at 450 nm and 570 nm was then immediately read.

### 1.12 Expression profile of CRC cell lines

Cancer cell lines were lysed and RNA was isolated using RNeasy Plus Micro Kit (QIAGEN). Next, reverse transcription RT-PCR was performed using SuperScript IV First-Strand Synthesis System (Thermo Fisher Scientific) to amplify cDNA. Obtained cDNA product was then used for amplification of 159-1240 bp and 60-1066 bp fragments (transcript variants 1 and 2) of TGFβR2 gene using gene specific primers TGFβR2 1k product_fwd 5'-ACTCCTGTGCAGCTTCCCTCGGC-3' [SEQ ID NO. 41] and TGFBR2 1k product_rvs 5'-GGCCTTATAGACCTCAGCAAAGCGACC-3' [SEQ ID NO. 42]. Amplified PCR products were visualized on 1% agarose gel, bands were cut out and DNA was purified using Zymoclean Gel DNA Recovery Kit (Zymo Research). Finally, the DNA was sequenced using Sanger sequencing technique (Eurofins).

### 1.13 Flow cytometry

The following antibodies (BioLegend) were used for staining at 1:100 dilutions unless stated otherwise: anti-mouse CD4-FITC (RM4-5), anti-mouse CD4-BV421 (L3T4, BD Pharmigen), anti-mouse CD3-PE (145-2C11), anti-mouse CD3-APC (145-2C11), anti-mouse IFNy-BV421 (XMG1.2), anti-mouse IFNy-PE (XMG1.2), anti-mTCRβ-chain-APC (H57-597), anti-human CD3-APC (SK7) and anti-human CD4-BV421 (OKT4). Magnetic beads anti-human CD8 (Microbeads 130-045-201) were used for CD8 depletion at 1:250 dilution (Miltenyi Biotec). FACSAria II (BD Bioscience) was used for sorting CD4⁺ responsive T cells, FACSAria F (BD Bioscience) was used to sort transduced cell lines. For analysis of cells, LSR-Fortessa was used (BD Bioscience).

### Example 2: Identification of DRB1^{∗}o4:o1-restricted TGFβR2(-1) peptide

Based on in silico pMHC binding prediction data (NetMHCIIpan 3.2 Server), the TGFβR2(-1) frameshift peptide gives suitable epitopes that can bind to HLA-DRB1^{∗}04:01, with one of the best predicted binding affinities being 25,2 nM (VALMSAMTTSSSQKN) [SEQ ID NO. 10] (Table 1). For this putative epitope, T cell receptors were generated by immunizing ABabDR4 mice with the TGFβR2(-1) peptide VALMSAMTTSSSQKN [SEQ ID NO. 10].

**Table 1. Peptide:MHC class II binding affinities of frameshift-derived epitopes for HLA-DRB1^{∗}04:01 allele predicted by prediction binding Servers NetMHCIIpan 3.2, NetMHCIIpan 4.0 and IEDB recommended 2.22. Sequence highlighted in bold letters is the one chosen for further experiments. The predicted output is given as IC50 (nM) units or as a rank. The lower the number, the higher is the affinity, the better the rank.**

| Frameshift peptide sequence: **SLVRLSSCVPVALMSAMTTSSSQKNITPAILTCC** [SEQ ID NO.9] | | | | |
|---|---|---|---|---|
| **NetMHCIIpan 3.2** | | | NetMHCIIpan 4.0 | IEDB recommended 2.22 |
| epitope sequence | peptide core [SEQ ID NO. 43] | IC50 (nM) | rank | IC50 (nM) |
| CVPVALMSAMTTSSS [SEQ ID NO. 44] | MSAMTTSSS | 47.2 | 21 | 51.0 |
| VPVALMSAMTTSSSQ [SEQ ID NO. 45] | MSAMTTSSS | 33.2 | 8.70 | 50.0 |
| PVALMSAMTTSSSQK [SEQ ID NO. 46] | MSAMTTSSS | 24.7 | 3.60 | 49.0 |
| **VALMSAMTTSSSQKN** [SEQ ID NO.9] | **MSAMTTSSS** | **25.2** | **3.50** | **49.0** |
| ALMSAMTTSSSQKNI [SEQ ID NO. 47] | MSAMTTSSS | 30.3 | 9.40 | 67.0 |
| LMSAMTTSSSQKNIT [SEQ ID NO. 48] | MSAMTTSSS | 43.4 | 25 | 153.0 |

### Example 3: Isolation of TGFβR2(-1)-reactive TCRs from ABabDR4 mice

We immunized ABabDR4 mice with the TGFβR2(-1) peptide VALMSAMTTSSSQKN [SEQ ID NO. 10]. Significant CD4⁺ T cell expansion was observed in immunized mice after restimulation of peripheral blood-derived T cells with TGFβR2(-1) peptide (Figure 1A, B). To isolate TGFβR2(-1)-reactive TCRs, we collected splenocytes from the most responsive mouse nr 1414 on day 10 after 3^{rd} immunization (Figure 1B). Following splenocytes restimulation in *vitro* for 4 hrs with TGFβR2(-1) peptide, we labelled responding T cells using Mouse IFNγ Secretion Assay KIT. Such labelled cells were quantitatively analysed and antigen-specific CD4⁺ T cell population was collected by flow cytometry. We collected 6 000 cells which stood for 1.22% of CD3⁺ lymphocytes (Figure 1C).

Three dominant TCRα and five dominant TCRβ chains were identified in collected antigen-specific CD4⁺ T cells (Figure 2A). Combinatorial expression of each out of the 3 TCRα with each of the 5 TCRβchains was performed, resulting in 15 different TCRαβ combinations that were transduced into human peripheral blood leukocytes (PBLs)-derived T cells (Figure 2B). To reduce cross pairing of identified TCRα/β with endogenous TCRα/β chains, their constant region was murinized (Sommermeyer *et al.,* 2010). All 15 TCRαβ combinations prepared were used for co-culture with BM14 cell line (naturally expressing HLA-DRB1^{∗}04:01) to determine functional TCRαβ combinations. Functional TCRαβ combinations are the ones recognizing the epitope presented on HLA-DRB1^{∗}04:01 molecules of antigen presenting target cells BM14, resulting in IFNy production upon peptide stimulation. Functional TCR pairs 1A 1B and 2A 3B were identified as reflected by the high IFNy amount measured in the supernatant by ELISA (Figure 23C). Functional TCRαβ chain combinations were cloned together into one expression vector and linked with P2A element for equal molarity expression (Leisegang *et al.,* 2008). Next, they were synthetized along with an amino acid codon optimization for *Homo sapiens* species for better expression in human T cells. Functional TCR pairs TCR 1A 1B and TCR 2A 3B were called TCR1414_1 and TCR1414_2, respectively.

### Example 4: Peptide sensitivity

One of the important feature of a TCR is its sensitivity to peptide concentration at which the TCR still recognizes the pMHC complex. To analyze peptide sensitivity, human CD4⁺ T cells transduced with TCR1414_1 or TCR1414_2 were co-cultured with BSM (HLA-DR4⁺) cells loaded with different concentrations of TGFβR2(-1) peptide. As a readout, IFNy concentration was measured by ELISA. Both investigated TCRs showed recognition up to 10⁻⁹ M TGFβR2(-1) peptide with IC50 values between 10 and 1 nM for TCR1414_1 and TCR1414_2, respectively (Figure 3).

### Example 5: TGFβR2(-1) neoantigen is naturally processed and presented

Immunization of ABabDR4 mice with TGFβR2(-1) peptide VALMSAMTTSSSQKN [SEQ ID NO. 10] shows its immunogenicity but does not reveal if the whole frameshift antigen is processed by endogenous endosomal-lysosomal antigen-processing compartments and presented on HLA-DRA/HLA-DRB1^{∗}04:01 molecules. To address the question whether the TGFβR2(-1) neoantigen is processed and presented, TCR1414_1/2-transduced PBLs-derived CD4⁺ T cells (Figure 4A) were co-cultured with antigen presenting BSM cells (HLA-DR4⁺) transduced with a TGFβR2(-1) minigene [SEQ ID NO. 43 and 44] encoding 47 amino acids containing TGFβR2(-1) frameshift peptide with HLA-DRB1^{∗}04:01-resctricted epitope of interest (Figure 4B). As a readout, IFNy and IL-2 concentrations were measured in the supernatant of the co-cultures by ELISA (Figure 5C). Results from Figure 4C show that both TCRs (TCR1414_ and TCR1414_2) recognize the BSM-TGFβR2(-1) cell line endogenously, expressing the full length TGFβR2(-1) frameshift region. This demonstrates that the given TGFβR2(-1) minigene has been processed further, generating HLA-DRB1^{∗}04:01-restricted peptide presented on BSM-TGFβR2(-1) cells surface. Control BSM-mCh cells do not express mutational TGFβR2(-1) frameshift peptide and therefore, T cells interacting with those cells do not produce IFNy or IL-2 if TGFβR2(-1) peptide is not externally loaded.

### TGFβR2(-1) minigene:

ATG GCT GCT TCT CCA AAG TGC ATT ATG AAG GAA AAA AAA AGC CTG GTG AGA CTT
M A A S P K C I M K E K K S L V R L

TCT TCA TGT GTT CCT GTA GCT CTG ATG AGT GCA ATG ACA ACA TCA TCT TCT CAG
S S C V P V A L M S A M T T S S S Q

AAG AAT ATA ACA CCA GCA ATC CTG ACT TGT TGC TAG
K N I T P A I L T C C stop

### [SEQ ID NO. 49]

### Example 6: Recognition of TGFβR2(-1) neoantigen naturally expressed in CRC cell lines.

We focused on analysis of two CRC cell lines, SW48 and HCT116, reported to naturally express the TGFβR2(-1) frameshift mutation (Woerner *et al.,* 2010). To confirm the deletion of one adenine in the TGFβR2 gene, which causes the frameshift peptide containing TGFβR2(-1) neoantigen, we isolated RNA from both CRC cell lines and performed RT-PCR, amplifying ~1 Kb fragment of the TGFβR2 gene. Electrophoresis in Figure 5A reveals two bands since PCR primers were designed to cover both splicing variants of the TGFβR2 gene. The purified ~1 Kb DNA fragments were purified from the gel and subjected to Sanger sequencing. Sequencing results showed the presence of the mutation (lack of one adenine), reflected in only 9 adenines being present in both CRC cell lines SW48 and HCT116 (Figure 5A). TGFβR2 wild type (10 adenines microsatellite) in FM3 melanoma cell line was used as a negative control in this experiment. Next, we checked if the TGFβR2(-1)-reactive TCR1414_1 and TCR1414_2 can recognize CRC cell lines SW48 and HCT116 that naturally express TGFβR2(-1) neoantigen. To do so, TCR1414_1/2-transduced human CD4⁺ T cells were co-cultured with SW48 and HCT116 cell lines transduced with HLA-DR4, due to the absence of the particular MHC II molecule in these CRC cells (Figure 5B). As expected, SW48-DR4 and HCT116-DR4 cell lines naturally expressing TGFβR2(-1) neoantigens were recognized by both TCR1414_1/2-transduced human CD4⁺ T cells, as reflected by IFNy release (Figure 5C).

### Example 7: ABabDR4-derived TGFβR2(-1)-specific TCRs showed no alloreactivity.

ABabDR4 mice used for generation of TGFβR2(-1)-specific TCRs lack more than HLA-DRA/DRB1^{∗}0401 human MHC molecules and therefore, there is a potential risk of alloreaction between these TCRs and other human HLAs. To investigate if generated TCRs are alloreactive, we transduced human CD4⁺ T cells with TCR1414_1 or TCR1414_2 and co-cultured them with a panel of 16 Epstein-Barr virus-transformed B-LCLs expressing different MHC I and MHC II molecules (Table 2). No alloreactivity was detected (Figure 6). BSM and BM14 LCLs naturally expressing HLA-DR4 were additionally loaded with TGFβR2(-1) peptide and used here as TCR-specificity control for co-culture with TCR1414_1/2-transduced CD4⁺ T cells.

**Table 2: HLA allotypes of the LCL obtained from IPD-IMGT/HLA - Human Leukocyte Antigen Sequence Database (Robinson et al., 2020).**

| **B-LCL** | **A*** | **B*** | **C*** | **DRA*** | **DRB1*** | **DRB3*** | **DRB4*** | **DRB5*** | **DQA1*** | **DQB1*** | **DPA1*** | **DPB1*** |
|---|---|---|---|---|---|---|---|---|---|---|---|---|
| **BSM** | 02:01 :01:01 | 15:01:01:01 | 03:04:01:01 | 01:01:01 | 04:01 :01 :01, 04:01 :01:02 | | 01:03:01 :01, 01:03:01 :03 | | 03:01:01 | 03:02:01 | 01:03:01 :01 | 02:01 :02:01 |
| **BM14** | 03:01:01:01 | 07:02:01 :01 | 07:02:01:03 | 01:01:01 | 04:01:01:02 | | 01:03:01:11 | | 03:01:01 | 03:02:01 | 01:03:01:04 | 04:01 :01:01 |
| **MT14B** | 31:01 :02:01 | 40:01:02:01 | 03:04:0101 | 01:01:01 | 04:04:01 | | 01:03:01:01 01:03:01 :03 | | 03:01:01 | 03:02:01 | 01:03:01 :05 | 04:02:01 :02 |
| **VAVY** | 01:01:01:01 | 08:01:01:01 | 07:01:01:01 | 01:02:02 | 03:01:01:01 | 01:01:02:01, 01:01:02:02 | | | 05:01 :01 :02 | 02:01:01:01 | 02:01 :02:02 | 01:01:01 |
| **KLO** | 01:01:01:01, 02:08 | 08:01:01:01, 50:01:01 | 06:02:01:02, 07:01:01:01 | | 03:01:01:01, 07:01:01:01 | 01:01:02:01, 01:01:02:02 | 01:03:01:01, 01:03:01:03 | | 02:01:01:01, 05:01:01:02 | 02:01:01, 02:02:01:01 | 01:03:01:02, 02:01:02:01 | 01:01:01, 104:01 |
| **KAS011** | 01:01 :01:01 | 37:01:01:01 | 06:02:01:01 | 01:01:01 | 16:01:01 | | | 02:02 | 01:02:02:01 | 05:02:0101 | 01:03:01 :02, 02:01:01 :02 | 04:01:01:01, 14:01:01:01 |
| **WIN** | 01:01 :01:01 | 57:01:01:01 | 06:02:01:01 | 01:01 | 07:01 :01 :01, 07:01:01:02 | | 01:01 | | 02:01 :01 :01, 02:01 :01 :02 | 02:02:01 :01, 03:03:02:01 | 01:03:01 :02, 02:01 :04 | 04:01 :01 :01, 13:01 :01:01 |
| **TISI** | 24:02:01 :01 | 35:08:01 | 04:01:01:06 | 01:01:01 | 11:03:01 | 02:02 | | | 05:05:01:01, 05:05:01:02 | 03:01:01 | 01:03:01 :05 | 04:02:01 :02 |
| **SPO** | 02:01:01:01 | 44:02:01:01 | 05:01:01:02 | 01:01 :01 | 11:01:01:01 | 02:02:01:02 | | | 01:02:02 | 05:02:01 | 01:03:01:01 | 02:01:02 |
| **HOR** | 33:03:01:02 | 44:02:01 :01 | 14:03 | 01:02:02 | 13:02:01 | 03:01:01 | | | 01:02:01:09 | 06:04:01 :01 | 01:03:01:01 | 04:01:01:26 |
| **AMALA** | 02:17:02:01 | 15:01:01:01 | 03:03:01:01 | 01:02:02 | 14:02:01 :01 | 01:01:02:05 | | | 05:03:01:01 | 03:01:01:01 | 01:03:01:15 | 04:02:01 :02 |
| **KAS116** | 24:02:01:01 | 51:01:01:03 | 12:03:01:01 | 01:01:01 | 01:01:01 | | | | 01:01:01:01 | 05:01 :01 | 02-01-01-01 | 13:01:01:01 |
| **DJS** | 02:01 :01:01 03:01:01:0 1 | 37:01:01, 35:01:01:02 | 04:01:01 :01, 06:02:01:01 | | 01:01:01, 16:01 :01 | | | 02:02 | 01:01:01:01, 01:02:02 | 05:02:01, 05:01:01:03 | 01:03:01 :02, 01:03:01 :05 | 04:01:01:01, 04:02:01 :02 |
| **WT24** | 02:01:01:01 | 27:05:02 | 02:02:02:01 | | 16:01:01 | | | 02:02 | | 05:02:01 | 01:03:01:03 | 03:01:01 |
| **KE** | 02:03:01, 11:01:01:01 | 38:02:01, 15:02:01 :01 | 08:01 :01, 07:02:01:01 | | 04:05:01, 08:14:01 | | 01:03:01:01 | | 03:03:01, 01:03:01 :05 | 04:01 :01, 06:01:01 | 02:02:02 | 02:02, 05:01:01 |
| **SA** | 24:02:01:01 | 07:02:01 :01 | 07:02:01:03 | 01:01:01 | 01:01:01 | | | | 01:01:01 | 05:01 :01 | 01:03:01 :05 | 04:02:01 :02 |

The features disclosed in the foregoing description, in the claims and/or in the accompanying drawings may, both separately and in any combination thereof, be material for realizing the invention in diverse forms thereof.

### REFERENCES

Brattain, M. G., Fine, W. D., Khaled, F. M., Thompson, J. & Brattain, D. E. Heterogeneity of malignant cells from a human colonic carcinoma. Cancer Res 41,1751-1756 (1981).
Chen, X., Poncette, L. & Blankenstein, T. Human TCR-MHC coevolution after divergence from mice includes increased nontemplate-encoded CDR3 diversity. J Exp Med 214, 3417-3433 (2017).
Cohen, C. J., Zhao, Y., Zheng, Z., Rosenberg, S. A. & Morgan, R. A. Enhanced antitumor activity of murine-human hybrid T-cell receptor (TCR) in human lymphocytes is associated with improved pairing and TCR/CD3 stability. Cancer Res 66, 8878-8886 (2006).
Engels, B., Cam, H., Schüler, T., Indraccolo, S., Gladow, M., Baum, C., Blankenstein, T. & Uckert, W. Retroviral vectors for high-level transgene expression in T lymphocytes. Hum Gene Ther 14, 1155-1168 (2003).
Ghani, K., Wang, X., de Campos-Lima, P.O., Olszewska, M., Kamen, A., Rivière, I. & Caruso, M. Efficient human hematopoietic cell transduction using RD114- and GALV-pseudotyped retroviral vectors produced in suspension and serum-free media. Hum Gene Ther 20, 966-974 (2009).
Jung, B., Staudacher, J. J. & Beauchamp, D. Transforming growth factor β superfamily signaling in development of colorectal cancer. Gastroenterology 152, 36-52 (2017).
Leibovltz, A., Stlnson, J. C., McCombs, W. B., McCoy, C. E., Mazur, K. C. & Mabry, N. D. Classification of human colorectal adenocarcinoma cell lines. Cancer Res 36, 4562-4569 (1976).
Leisegang, M., Engels, B., Meyerhuber, P., Kieback, E., Sommermeyer, D., Xue, S.-A., Reuß, S., Stauss, H. & Uckert, W. Enhanced functionality of T cell receptor-redirected T cells is defined by the transgene cassette. J Mol Med 86, 573-583 (2008).
Li, L.-P., Lampert, J. C., Chen, X., Leitao, C., Popović, J., Müller, W. & Blankenstein, T. Transgenic mice with a diverse human T cell antigen receptor repertoire. Nat Med 16, 1029-1034 (2010).
Maby, P., Tougeron, D., Hamieh, M., Mlecnik, B., Kora, H., Bindea, G., Angell, H. K., Fredriksen, T., Elie, N., Fauquembergue, E., Drouet, A., Leprince, J., Benichou, J., Mauillon, J., Le Pessot, F., Sesboue, R., Tuech, J.-J., Sabourin, J.-C., Michel, P., Frebourg, T., Galon, J. & Latouche, J.-B. Correlation between density of CD8+ T-cell infiltrate in microsatellite unstable colorectal cancers and frameshift mutations: a rationale for personalized immunotherapy. Cancer Res 75, 3446-3455 (2015).
Markowitz, S., Wang, J., Myeroff, L., Parsons, R., Sun, L., Lutterbaugh, J., Fan, R., Zborowska, E., Kinzler, K., Vogelstein, B. & et, al. Inactivation of the type II TGF-beta receptor in colon cancer cells with microsatellite instability. Science 268, 1336-1338 (1995).
Principe, D. R., Doll, J. A., Bauer, J., Jung, B., Munshi, H. G., Bartholin, L., Pasche, B., Lee, C. & Grippo, P. J. TGF-: Duality of Function Between Tumor Prevention and Carcinogenesis. J Natl Cancer Inst 106, djt369-djt369 (2014).
Principe, D. R., DeCant, B., Staudacher, J., Vitello, D., Mangan, R. J., Wayne, E. A., Mascariñas, E., Diaz, A. M., Bauer, J., McKinney, R. D., Khazaie, K., Pasche, B., Dawson, D. W., Munshi, H. G., Grippo, P. J. & Jung, B. Loss of TGFβ signaling promotes colon cancer progression and tumor-associated inflammation. Oncotarget 8, 3826-3839 (2016).
Robinson, J., Barker, D. J., Georgiou, X., Cooper, M. A., Flicek, P. & Marsh, S. G. E. IPD-IMGT/HLA Database. Nucleic Acids Res 48, D948-D955 (2020).
Saeterdal, I., Bjorheim, J., Lislerud, K., Gjertsen, M. K., Bukholm, I. K., Olsen, O. C., Nesland, J. M., Eriksen, J. A., Moller, M., Lindblom, A. & Gaudernack, G. Frameshift-mutation-derived peptides as tumor-specific antigens in inherited and spontaneous colorectal cancer. Proc Natl Acad Sci 98, 13255-13260 (2001).
Schwitalle, Y., Kloor, M., Eiermann, S., Linnebacher, M., Kienle, P., Knaebel, H. P., Tariverdian, M., Benner, A. & von Knebel Doeberitz, M. Immune response against frameshift-induced neopeptides in HNPCC patients and healthy HNPCC mutation carriers. Gastroenterology 134, 988-997 (2008).
Sommermeyer, D. & Uckert, W. Minimal amino acid exchange in human TCR constant regions fosters improved function of TCR gene-modified T cells. J Immunol 184, 6223-6231 (2010).
Tougeron, D., Fauquembergue, E., Rouquette, A., Le Pessot, F., Sesboüé, R., Laurent, M., Berthet, P., Mauillon, J., Di Fiore, F., Sabourin, J.-C., Michel, P., Tosi, M., Frébourg, T. & Latouche, J.-B. Tumor-infiltrating lymphocytes in colorectal cancers with microsatellite instability are correlated with the number and spectrum of frameshift mutations. Mod Pathol 22,1186-1195 (2009).
Tran E, Robbins PF, Rosenberg SA. 'Final common pathway' of human cancer immunotherapy: targeting random somatic mutations._Nat Immunol. 2017 Feb 15;18(3):255-262. doi: 10.1038/ni.3682.
Woerner, S. M., Yuan, Y. P., Benner, A., Korff, S., von Knebel Doeberitz, M. & Bork, P. SelTarbase, a database of human mononucleotide-microsatellite mutations and their potential impact to tumorigenesis and immunology. Nucleic Acids Res 38, D682-D689 (2010).

## Claims

1. An isolated *antigen binding protein (ABP)* which specifically binds to a Transforming Growth Factor β Receptor 2 (TGFβR2), preferably a mutated TGFβR2, derived antigenic peptide, or a variant thereof,
wherein the isolated ABP comprises at least one complementarity determining region (CDR) being a CDR3 having an amino acid sequence with at least 80% sequence identity to, or having no more than three or two, preferably no more than one amino acid substitution(s), deletion(s) or insertion(s) compared to, a sequence selected from SEQ ID Nos: 11, 13, 15, and 17.

2. The isolated ABP of claim 1, wherein said ABP is capable of specifically and/or selectively binding to an antigenic peptide derived from a frameshift variant of TGFβR2,
preferably wherein the frameshift variant of TGFβR2 is derived from a sequence comprising a frameshift mutation in the wild type TGFβR2 gene sequence according to SEQ ID No: 1 or 2, more preferably wherein the frameshift mutation in TGFβR2 is a deletion or an addition of one nucleotide.

3. The isolated ABP of claim 2, wherein the frame-shift mutation is a deletion or an addition of one nucleotide in a microsatellite region of the TGFβR2 gene sequence according to SEQ ID No: 1 or 2,
preferably wherein the microsatellite region is a polyadenine sequence, such as a repeat of adenines located in exon 3 of the wild type TGFβR2 gene sequence according to SEQ ID No: 1 or 2, for example a repeat of adenines within the sequence comprising nucleotide numbers 709 to 718 of the wild type TGFβR2 gene sequence according to SEQ ID No: 1 or 2, and wherein the frame-shift mutation is a deletion or an addition of one adenine.

4. The isolated ABP of claim 2 or 3, wherein the frame-shift mutation causes a shift of the open reading frame of the wild type TGFβR2 gene sequence according to SEQ ID No: 1 or 2 due to the deletion or addition of one nucleotide,
preferably wherein the frameshift variant of TGFβR2 comprises an amino acid at position 128 or 129 according to the numbering in the wild type TGFβR2 amino acid sequence according to SEQ ID No: 5 or 6 that differs from the amino acid at position 128 or 129 of the wild type TGFβR2 amino acid sequence according to SEQ ID No: 5 or 6, more preferably wherein the frameshift variant of TGFβR2 comprises an amino acid sequence according to SEQ ID No: 10.

5. The isolated ABP of any one of claims 2 to 4, wherein the frame-shift mutation is a deletion of one nucleotide within the sequence comprising nucleotide numbers 709 to 718 of the wild type TGFβR2 gene sequence according to SEQ ID No: 1 or 2, preferably wherein the deletion of one nucleotide is a deletion of one adenine,
and/or wherein the ABP is an antibody, or an antigen binding derivative or fragment thereof, or, preferably, is a T cell receptor (TCR), or an antigen binding derivative or fragment thereof,
and/or wherein the ABP binds to a human leucocyte antigen (HLA) presented antigenic peptide, wherein said HLA is preferably a HLA type DR4, preferably wherein the ABP forms a specific complex with the MHC and the antigenic peptide,
and/or wherein the ABP is humanized, chimerized and/or murinized.

6. The isolated ABP of any one of the preceding claims, wherein the ABP specifically and/or selectively binds to an epitope having the amino acid sequence selected from an MHC presented peptide of a frameshift variant of TGFβR2, preferably wherein the peptide comprises a frame-shift mutation in TGFβR2, and most preferably a peptide comprising SEQ ID No: 10,
and/or wherein the ABP is an α/β-TCR, or an antigen binding derivative or a fragment thereof, or wherein the ABP is a γ/δ-TCR, or an antigen binding derivative or a fragment thereof,
and/or wherein the isolated ABP is **characterized in that** the ABP is of human origin and specifically and/or selectively recognizes a TGFβR2 frameshift antigenic peptide,
and/or wherein said ABP is capable of inducing an immune response in a subject, optionally wherein the immune response is **characterized by** an increase in interferon (IFN) γ levels.

7. The isolated ABP of any one of the preceding claims, comprising
a TCR α or γ chain; and/or
a TCR β or δ chain;
wherein the TCR α or γ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID Nos: 11 and 15; and/or
wherein the TCR β or δ chain comprises a CDR3 having an amino acid sequence with at least 80% sequence identity to, or having no more than three or two, preferably no more than one amino acid substitution(s), deletion(s) or insertion(s) compared to, a sequence selected from SEQ ID Nos: 13 and 17.

8. The isolated ABP of any one of the preceding claims, comprising a TCR variable chain region having at least 80% sequence identity to, or having no more than 20, preferably no more than 15, more preferably no more than 10, 5, three or two, preferably no more than one amino acid substitution(s), deletion(s) or insertion(s) compared to, an amino acid sequence selected from SEQ ID Nos. 12, 14, 16, and 18,
and/or comprising a binding fragment of a TCR, and wherein said binding fragment comprises a CDR1 to CDR3, optionally selected from the CDR1 to CDR3 sequences having the amino acid sequences of SEQ ID Nos. 11, 13, 15, and 17; each CDR independently having no more than three or two, preferably no more than one amino acid substitution(s), deletion(s) or insertion(s) compared to these sequences.

9. The isolated ABP of any one of the preceding claims, wherein the ABP is a TCR, or an antigen-binding fragment or derivative thereof, comprising at least one TCR α and one TCR β chain sequence,
wherein said TCR α chain sequence comprises a variable region sequence having the amino acid sequence of SEQ ID No: 12, and
wherein said TCR β chain sequence comprises a variable region sequence having the amino acid sequence of SEQ ID No: 14; or
wherein said TCR α chain sequence comprises a variable region sequence having the amino acid sequence of SEQ ID No: 16, and
wherein said TCR β chain sequence comprises a variable region sequence having the amino acid sequence of SEQ ID No: 18;
each variable domain sequence having no more than 20, preferably no more than 15, more preferably no more than 10, 5, three or two, preferably no more than one amino acid substitution(s), deletion(s) or insertion(s) compared to these sequences.

10. The isolated ABP of any one of the preceding claims, wherein the ABP is a TCR, or an antigen-binding fragment or derivative thereof, further comprising a TCR constant region, preferably a human TCR constant region sequence.

11. An isolated *nucleic acid* encoding for an ABP of any one of claims 1 to 10,
preferably comprising a nucleic acid sequence having at least 80% sequence identity to, or having no more than 20, preferably no more than 15, more preferably no more than 10, 5, three or two, preferably no more than one nucleic acid substitution(s), deletion(s) or insertion(s) compared to, a nucleic acid sequence encoding for a polypeptide selected from SEQ ID Nos: 12, 14, 16, and 18.

12. A recombinant *vector* comprising a nucleic acid of claim 11.

13. A recombinant *host cell* comprising an ABP of any one of claims 1 to 10, or a nucleic acid of claim 11, or a vector of claim 12,
preferably wherein the host cell is a lymphocyte, more preferably a T lymphocyte or T lymphocyte progenitor, even more preferably a CD4 or a CD8 positive T-cell, most preferably a CD4 positive T-cell.

14. A *pharmaceutical composition* comprising
the ABP of any one of claims 1 to 10, or a nucleic acid of claim 11, or a vector of claim 12, or the host cell of claim 13, and
a pharmaceutical acceptable carrier, stabilizer and/or excipient;
preferably wherein the pharmaceutical composition comprises at least two different ABP of any one of claims 1 to 10.

15. A compound or composition *for use in medicine,* wherein the compound or composition is selected from an ABP of any one of claims 1 to 10, or a nucleic acid of claim 11, or a vector of claim 12, or a host cell of claim 13, or a pharmaceutical composition of claim 14,
preferably for use is in the treatment of a proliferative disorder, more preferably cancer, such as a cancer positive for the expression of a TGFβR2 frameshift peptide.

16. The compound or composition for use of claim 15, wherein the treatment comprises immune therapy, preferably adoptive autologous or heterologous T-cell therapy.
wherein, preferably, the T-cell therapy involves the use of a cell comprising the ABP or a nucleic acid of any one of the preceding claims.

17. A *method of manufacturing a* TGFβR2 frameshift peptide-specific antigen recognizing construct expressing *cell line,* preferably a TGFβR2(-1) frameshift peptide-specific antigen recognizing construct expressing cell line, comprising
(a) providing a suitable host cell,
(b) providing a genetic construct comprising a coding sequence encoding the ABP according to any of claims 1 to 10,
(c) introducing into said suitable host cell said genetic construct, and
(d) expressing said genetic construct by said suitable host cell,
wherein said ABP is preferably an alpha/beta TCR, gamma/delta TCR, or a TCR variant, such as a single chain TCR (scTCR),
and/or wherein the suitable host cell is preferably a T-cell,
more preferably a T cell obtained from a patient suffering from a disorder associated with a TGFβR2 frameshift peptide expression, more preferably with a TGFβR2(-1) frameshift peptide expression, such as even more preferably a cancer, for example colorectal cancer (CRC).
